(19) Europäisches Patentamt
European Patent Office
Office européen des brevets

(11) EP 3 970 745 B1

(12) EUROPEAN PATENT SPECIFICATION

(45) Date of publication and mention of the grant of the patent:
**20.05.2026 Bulletin 2026/21**

(21) Application number: **20847524.4**

(22) Date of filing: **27.07.2020**

(51) International Patent Classification (IPC):
***A61K 31/357*** *(2006.01)* ***A61K 9/1271*** *(2025.01)*
***A61K 39/395*** *(2006.01)* ***A61P 35/04*** *(2006.01)*
***C07K 16/28*** *(2006.01)* ***A61K 39/00*** *(2006.01)*

(52) Cooperative Patent Classification (CPC):
(C-Sets available)
**A61K 31/357; A61K 9/1271; A61K 39/39541; C07K 16/2818;** A61K 2039/505; A61P 35/04
(Cont.)

(86) International application number:
**PCT/JP2020/028663**

(87) International publication number:
**WO 2021/020336 (04.02.2021 Gazette 2021/05)**

(54) **COMBINATION OF A LIPOSOMAL COMPOSITION OF ERIBULIN WITH A PD-1 ANTAGONIST FOR USE IN TREATING TUMOURS**

KOMBINATION EINER LIPOSOMALEN ZUSAMMENSETZUNG VON ERIBULIN MIT EINEM PD-1-ANTAGONISTEN ZUR VERWENDUNG BEI DER BEHANDLUNG VON TUMOREN

COMBINAISON D’UNE COMPOSITION LIPOSOMALE D’ÉRIBULINE ET D’UN ANTAGONISTE DE PD-1 POUR UNE UTILISATION DANS LE TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.07.2019 JP 2019138041**
**31.03.2020 US 202016835719**

(43) Date of publication of application:
**23.03.2022 Bulletin 2022/12**

(73) Proprietor: **Eisai R&D Management Co., Ltd.**
**Tokyo 112-8088 (JP)**

(72) Inventors:
- **SEMBA Taro**
  **Tsukuba-shi, Ibaraki 300-2635 (JP)**
- **FUNAHASHI Yasuhiro**
  **Tsukuba-shi, Ibaraki 300-2635 (JP)**
- **SUZUKI Takuya**
  **Tokyo 112-8088 (JP)**

(74) Representative: **Hoffmann Eitle**
**Patent- und Rechtsanwälte PartmbB**
**Arabellastraße 30**
**81925 München (DE)**

(56) References cited:
**WO-A1-2010/113983** **WO-A1-2010/113984**
**WO-A1-2017/188350** **WO-A1-2018/071792**
**WO-A1-2018/071792** **JP-A- 2018 508 516**
**US-A1- 2018 071 247** **US-A1- 2019 010 232**
**US-A1- 2022 389 110**

- **SM TOLANEY, K KALINSKY, V KAKLAMANI, C SAVULSKY, M OLIVO, G AKTAN, PA KAUFMAN, D XING, A ALMONTE, S MISIR, V KARANTZA, S DIAB: "ABSTRACT PD6-13 :Phase Ib/2 study to evaluate eribulin mesylate in combination with pembrolizumab in patients with metastatic triple-negative breast cancer", CANCER RESEARCH, vol. 78, no. 4, 5 December 2017 (2017-12-05), XP055749240, ISSN: 1538-7445**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ANONYMOUS: "History of Changes for Study: NCT04078295 A Study of E7389 liposomal Formulation (E7389-LF) Plus Nivolumab in Participants With Solid Tumor", 2 September 2019 (2019-09-02), pages 1 - 5, XP055879022, Retrieved from the Internet <URL:https://clinicaltrials.gov/ct2/history/NCT04078295?V_1=View#StudyPageTop>**

Remarks:

The file contains technical information submitted after the application was filed and not included in this specification

(52) Cooperative Patent Classification (CPC): (Cont.)

C-Sets

**A61K 31/357, A61K 2300/00;**
**A61K 39/39541, A61K 2300/00**

## Description

## Technical Field

The present invention relates to a pharmaceutical composition for use in treating tumor.

Background Art

Eribulin represented by formula (I) is used as a therapeutic agent for breast cancer and soft tissue tumor.

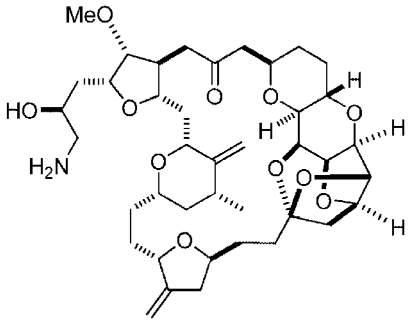

(I)

Patent Literature 1 discloses Eribulin or a pharmaceutically acceptable salt thereof and a method of producing the same. Patent Literatures 2 and 3 disclose methods for producing Eribulin and Eribulin mesylate, which is a mesylate (methanesulfonate) thereof. Patent Literature 4 discloses a method of inhibiting growth of cancer in a patient by administering Eribulin or a pharmaceutically acceptable salt thereof to the patient. Patent Literature 5 discloses a method of treating cancer in a patient by administering Eribulin or a pharmaceutically acceptable salt thereof to the patient in combination with a certain second anticancer agent. Patent Literature 6 discloses a method of treating cancer in a patient by administering Eribulin or a pharmaceutically acceptable salt thereof to the patient in combination with a second therapeutic approach. Patent Literatures 7 and 8 disclose liposomal compositions comprising Eribulin mesylate. Patent Literature 9 discloses a method for treating breast cancer, comprising administering a combination of Eribulin or a pharmaceutically acceptable salt thereof and a programmed cell death 1 protein (PD-1) antagonist.

PD-1 is recognized as an important factor in maintenance of immunoregulation and peripheral tolerance. PD-1 is moderately expressed in naive T cells, B cells, and NK T cells, and upregulated by T/B cell receptor signal transduction in lymphocytes, monocytes, and myeloid cells (Non Patent Literature 1). Meanwhile, PD-L1 is expressed in various cancer cells or T/B cells, macrophages, mDCs, plasmacytoid DCs: (pDCs), bone marrow mast cells, and the like.

The two known PD-1 ligands PD-L1 (B7-H1) and PD-L2 (B7-DC) are expressed in human cancer occurring in various tissues. For example, in a large amount of sample sets of ovarian cancer, renal cancer, colorectal cancer, pancreatic cancer, liver cancer, and melanoma, the PD-L1 expression has been shown to correlate with poor prognosis and decreased overall survival, regardless of subsequent treatment (Non Patent Literatures 2 to 13). Similarly, it was found that the PD-1 expression in tumor-infiltrating lymphocytes is characteristic of functionally impaired T cells in breast cancer and melanoma (Non Patent Literatures 14 to 15) and correlates with poor prognosis in kidney cancer (Non Patent Literature 16). Therefore, it has been proposed to block the immunosuppression mechanism that cancer cells bring, such as the interaction of tumor cells expressing PD-L1 with T cells expressing PD-1, and thereby bring the immune response to tumor.

Several monoclonal antibodies that inhibit the interaction between PD-1 and either or both of PD-1 ligands PD-L1 and PD-L2 are under clinical development for treating cancer. It has been proposed that the efficacy of such antibodies may be increased when administered in combination with another approved or experimental cancer therapy, for example, radiation, surgery, a chemotherapeutic agent, a targeted therapy, an agent that inhibits another signaling pathway that is dysregulated in tumor, and another immunostimulant.

## Citation List

## Patent Literature

Patent Literature 1: WO 99/65894
Patent Literature 2: WO 2005/118565

Patent Literature 3: WO 2011/094339
Patent Literature 4: U.S. Patent No. 6469182
Patent Literature 5: U.S. Application Publication No. 2006/104984
Patent Literature 6: U.S. Patent No. 6653341
Patent Literature 7: WO 2010/113984
Patent Literature 8: WO 2017/188350
Patent Literature 9: WO 2016/141209

**Non Patent Literature**

Non Patent Literature 1: Sharpe, A.H, Wherry, E.J., Ahmed R., and Freeman G.J., The function of programmed cell death 1 and its ligands in regulating autoimmunity and infection. Nature Immunology (2007); 8: 239-245.
Non Patent Literature 2: Dong H et al., Tumor-associated B7-H1 promotes T-cell apoptosis: a potential mechanism of immune evasion. Nat Med. 2002 Aug; 8(8): 793-800.
Non Patent Literature 3: Yang et al., PD-1 interaction contributes to the functional suppression of T-cell responses to human uveal melanoma cells in vitro. Invest Ophthalmol Vis Sci. 2008 Jun; 49(6 (2008): 49: 2518-2525.
Non Patent Literature 4: Ghebeh et al., The B7-H1 (PD-L1) T lymphocyte-inhibitory molecule is expressed in breast cancer patients with infiltrating ductal carcinoma: correlation with important high-risk prognostic factors. Neoplasia (2006) 8: 190-198.
Non Patent Literature 5: Hamanishi J et al., Programmed cell death 1 ligand 1 and tumor-infiltrating CD8+ T lymphocytes are prognostic factors of human ovarian cancer. Proceeding of the National Academy of Sciences (2007): 104: 3360-3365.
Non Patent Literature 6: Thompson RH et al., Significance of B7-H1 overexpression in kidney cancer. Clinical genitourin Cancer (2006): 5: 206-211.
Non Patent Literature 7: Nomi, T. Sho, M., Akahori, T., et al., Clinical significance and therapeutic potential of the programmed death- 1 ligand/programmed death-1 pathway in human pancreatic cancer. Clinical Cancer Research (2007) ;13: 2151-2157.
Non Patent Literature 8: Ohigashi Y et al., Clinical significance of programmed death-1 ligand-1 and programmed death-1 ligand 2 expression in human esophageal cancer. Clin. Cancer Research (2005): 11: 2947-2953.
Non Patent Literature 9: Inman et al., PD-L1 (B7-H1) expression by urothelial carcinoma of the bladder and BCG-induced granulomata: associations with localized stage progression. Cancer (2007): 109: 1499-1505.
Non Patent Literature 10: Shimauchi T et al., Augmented expression of programmed death-1 in both neoplasmatic and nonneoplastic CD4+ T-cells in adult T-cell Leukemia/ Lymphoma. Int. J. Cancer (2007): 121: 2585-2590.
Non Patent Literature 11: Gao et al., Overexpression of PD-L1 significantly associates with tumor aggressiveness and postoperative recurrence in human hepatocellular carcinoma. Clinical Cancer Research (2009) 15: 971-979.
Non Patent Literature 12: Nakanishi J., Overexpression of B7-H1 (PD-L1) significantly associates with tumor grade and postoperative prognosis in human urothelial cancers. Cancer Immunol Immunother. (2007) 56: 1173- 1182.
Non Patent Literature 13: Hino et al., Tumor cell expression of programmed cell death-1 is a prognostic factor for malignant melanoma. Cancer (2010): 116: 1757-1766.
Non Patent Literature 14: Ghebeh H., Foxp3+ tregs and B7-H1+/PD-1+ T lymphocytes co-infiltrate the tumor tissues of high-risk breast cancer patients: implication for immunotherapy. BMC Cancer. 2008 Feb 23; 8:57.
Non Patent Literature 15: Ahmadzadeh M. et al., Tumor antigen-specific CD8 T cells infiltrating the tumor express high levels of PD-1 and are functionally impaired. Blood (2009) 114: 1537-1544.
Non Patent Literature 16: Thompson RH et al., PD-1 is expressed by tumor infiltrating cells and is associated with poor outcome for patients with renal carcinoma. Clinical Cancer Research (2007) 15: 1757-1761.

**Summary of Invention**

**Technical Problem**

The present invention is directed to provide a new pharmaceutical composition for use in treating tumor.

**Solution to Problem**

The present inventors have studied diligently and, as a result, found that the combined administration of a liposomal composition comprising Eribulin or a pharmaceutically acceptable salt thereof and a PD-1 antagonist exhibits

unexpected antitumor effect, thereby completing the present invention.

The present invention is as defined in the appended claims.

## Advantageous Effects of Invention

The combined administration of a liposomal composition comprising Eribulin or a pharmaceutically acceptable salt thereof and a PD-1 antagonist exhibits unexpected antitumor effect.

## Brief Description of The Drawings

FIG. 1 is a graph illustrating effect of treatment with a combination of a liposomal formulation comprising Eribulin mesylate at a dose of 0.1 mg/kg and an anti-PD-1 antibody on tumor growth.

FIG. 2 is a graph illustrating effect of treatment with a combination of a liposomal formulation comprising Eribulin mesylate at a dose of 0.1 mg/kg and an anti-PD-1 antibody on Tx5.

FIG. 3 is a graph illustrating effect of treatment with a combination of a liposomal formulation comprising Eribulin mesylate at a dose of 0.3 mg/kg and an anti-PD-1 antibody on tumor growth.

FIG. 4 is a graph illustrating effect of treatment with a combination of a liposomal formulation comprising Eribulin mesylate at a dose of 0.3 mg/kg and an anti-PD-1 antibody on Tx5.

## Description of Embodiments

Embodiments of the present disclosure will be described below.

The liposomal compositions in the present disclosure comprises Eribulin or a pharmaceutically acceptable salt thereof (hereinafter referred to as " Eribulin or the like").

In the present disclosure, the "pharmaceutically acceptable salt" may be either an inorganic acid salt or an organic acid salt and is not particularly limited, as long as it forms a salt with Eribulin, and examples thereof include hydrochloride, sulfate, citrate, hydrobromide, hydroiodide, nitrate, bisulfate, phosphate, superphosphate, isonicotinate, acetate, lactate, salicylate, tartrate, pantothenate, ascorbate, succinate, maleate, fumarate, gluconate, saccharinate, formate, benzoate, glutamate, mesylate (methanesulfonate), ethanesulfonate, benzenesulfonate, p-toluenesulfonate, and pamoate. In one embodiment, the pharmaceutically acceptable salts are hydrochloride, sulfate, acetate, phosphate, citrate, and mesylate. In a particular embodiment, the pharmaceutically acceptable salt is mesylate.

The pharmaceutically acceptable salt of Eribulin may be a salt of Eribulin and aluminum, calcium, lithium, magnesium, sodium, zinc, or diethanolamine.

In the present disclosure, examples of Eribulin or the like include Eribulin mesylate.

Eribulin or the like is a compound or a salt thereof described in Patent Literature 1 or U.S. Patent No. 6214865 and has pharmacological activities including antitumor and antimitotic activities. Patent Literature 1 discloses that Eribulin or the like has, as an antitumor agent, anti-tumor activity against melanoma, fibrosarcoma, monocytic leukemia, colon cancer, ovarian cancer, breast cancer, osteosarcoma, prostate cancer, lung cancer, and ras-transformed fibroblasts. Eribulin or the like is obtained by a method of production described in Patent Literatures 1 to 3.

In the present disclosure, the "liposome" means a closed microvesicle having an inner phase surrounded by a lipid bilayer. The liposomes include small unilamellar liposomes (SUVs: small unilamellar vesicles), large unilamellar liposomes (LUVs: large unilamellar vesicles), further large unilamellar liposomes (GUVs: giant unilamellar vesicles), multilamellar liposomes having a plurality of concentric membranes (MLVs: multi lamellar vesicles), liposomes having a plurality of non-concentric, irregular membranes (MVVs: multivesicular vesicles), and the like.

In the present disclosure, the "liposomal inner phase" means an aqueous region surrounded by a liposomal lipid bilayer and is used synonymously with an "inner aqueous phase" and a "liposomal inner aqueous phase". The "liposomal outer phase" means a region that is not surrounded by a liposomal lipid bilayer (that is, the region except the inner phase and the lipid bilayer) when the liposome is dispersed in a liquid.

In the present disclosure, the "liposomal composition" means a composition comprising a liposome and further comprising Eribulin or the like in the liposomal inner phase. In the present disclosure, the liposomal composition includes solid and liquid compositions.

In the present disclosure, the "liposomal dispersion liquid" means a composition comprising a liposome in which Eribulin or the like is not yet encapsulated into the liposomal inner phase.

In the present disclosure, the "liposomal preparatory liquid" means a composition comprising a liposome in which an adjustment of the liposome outer phase in order to encapsulate Eribulin or the like into the liposome inner phase is not yet performed.

[Lipid]

In one embodiment, the liposome preferably comprises a phospholipid and/or phospholipid derivative as a membrane component.

Examples of the phospholipid and/or phospholipid derivative include phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerolphosphate, 1,2-dimyristoyl-1,2-deoxyphosphatidyl choline, plasmalogen, and phosphatidate.

The phospholipid and/or phospholipid derivative may be one or a combination of two or more of these.

Fatty acid residues in the phospholipid and/or phospholipid derivative are not particularly limited, and examples thereof include saturated or unsaturated fatty acid residues having 12 to 20 carbon atoms, and specific examples thereof include acyl groups derived from fatty acids such as lauric acid, myristic acid, palmitic acid, stearic acid, oleic acid, and linoleic acid. As the phospholipid and/or phospholipid derivative, a phospholipid derived from a natural product such as egg yolk lecithin and soy lecithin, and partially hydrogenated egg yolk lecithin, (fully) hydrogenated egg yolk lecithin, partially hydrogenated soy lecithin, and (fully) hydrogenated soy lecithin, in which unsaturated fatty acid residues are partially or fully hydrogenated, or the like may be used.

The blending amount (molar fraction) of the phospholipid and/or phospholipid derivative, used in the preparation of the liposome is not particularly limited, and is, in one embodiment, 10 to 80% and, in a particular embodiment, 30 to 60% based on the total ribosomal membrane components.

In the present disclosure, the liposome may comprise, as a membrane component, a sort of sterol such as cholesterol and cholestanol and a sort of fatty acid having a saturated or unsaturated acyl group having 8 to 22 carbon atoms as a membrane stabilizing agent, and an antioxidant such as $\alpha$-tocopherol, besides the phospholipid and/or phospholipid derivative.

The blending amount (molar fraction) of the sterol, used in the preparation of the liposome is not particularly limited, and is, in one embodiment, 1 to 60%, 10 to 50%, or 30 to 50% based on the total liposomal membrane components.

The blending amount (molar fraction) of the fatty acid is not particularly limited, and is, in one embodiment, 0 to 30%, 0 to 20% or 0 to 10% based on the total liposomal membrane components.

The blending amount (molar fraction) of the antioxidant is not particularly limited, as long as an amount that provides the antioxidant effect is added, and it is, in one embodiment, 0 to 15%, 0 to 10%, or 0 to 5% based on the total liposomal membrane components.

In the present disclosure, the liposome may comprise a functional lipid or a modified lipid as a membrane component.

Examples of the functional lipid include a blood-retaining lipid derivative, a temperature change-sensitive lipid derivative, and a pH-sensitive lipid derivative.

Examples of the modified lipid include a PEGylated lipid, a glycolipid, an antibody-modified lipid, and a peptide-modified lipid.

Examples of the blood-retaining lipid derivative include polyethylene glycol derivatives (such as methoxy polyethylene glycol condensates) such as condensation products of phosphoethanolamine and methoxy polyethylene glycol: N-{carbonyl-methoxy polyethylene glycol-2000}-1,2-dipalmitoyl-sn-glycero-3-phosphoethanolamine, N-{carbonyl-methoxy polyethylene glycol-5000}-1,2-dipalmitoyl-sn-glycero-3-phosphoethanolamine, N-{carbonyl-methoxy polyethylene glycol-750}-1,2-distearoyl-sn-glycero-3-phosphoethanolamine, N-{carbonyl-methoxy polyethylene glycol-2000}-1,2-distearoyl-sn-glycero-3-phosphoethanolamine, (MPEG2000-distearoylphosphatidylethanolamine), and N-{carbonyl-methoxy polyethylene glycol-5000}-1,2-distearoyl-sn-glycero-3-phosphoethanolamine.

The blending amount (molar fraction) of the blood-retaining lipid derivative, used in the preparation of the liposome, is not particularly limited, and is, in one embodiment, 0 to 50%, 0 to 30%, or 0 to 20% based on the total liposomal membrane components.

Examples of the temperature change-sensitive lipid derivative include dipalmitoylphosphatidylcholine. By including the temperature change-sensitive lipid derivative in a liposome, it becomes possible to disrupt the liposome at a particular temperature, to change the surface properties of the liposome, and the like. Furthermore, by combining it with heating of a target site such as tumor, it becomes possible to disrupt the liposome at the target site and have an active compound released at the target site, and the like.

Examples of the pH-sensitive lipid derivative include dioleoylphosphatidylethanolamine. By including the pH-sensitive lipid derivative in a liposome, it becomes possible to promote membrane fusion of the liposome and an endosome when the liposome is taken in a cell by endocytosis and improve the delivery of the active compound to the cytoplasm, and the like.

Examples of the glycolipid, antibody-modified lipid, and peptide-modified lipid include lipids linked to a sugar, an antibody, or a peptide having an affinity for a target cell or a target tissue. Using a modified lipid allows to deliver the liposome actively to the target cell or the target tissue.

The composition of membrane components for liposome having a practically acceptable level of membrane permeability can be set by a person skilled in the art as appropriate depending on the active compound, the target tissue, and the like (see Hiroshi Kikuchi et al. "Liposome I -How to prepare and assay-" Cell technology (1983) 2 (9): pp. 1136-1149 and the references cited in the reference, and the like). The liposomal composition may be used not only in targeting at a target tissue such as solid cancer, but also in the delivery of an active compound to blood cancer or the like.
The liposomal membrane components include, in one embodiment, phospholipid, cholesterol, and a methoxy polyethylene glycol condensation product.

[Liposomal composition]

In the liposomal composition in the present disclosure, Eribulin or the like is encapsulated in a liposome having a lipid membrane. In the liposomal composition, Eribulin or the like may be distributed in the lipid bilayer.
The liposomal composition according to the present disclosure can be obtained by a method described in Patent Literature 7.
If the liposomal composition is solid, it may be dissolved or suspended in a certain solvent described below to prepare a liquid liposomal composition. If the liposomal composition is in the form of frozen solid, it may be thawed by leaving it at room temperature or the like to prepare a liquid liposomal composition.
The liposomal composition according to the present disclosure is not limited, as long as it comprises (1) Eribulin or the like. The liposomal composition according to the present disclosure may further comprise (2) at least one ammonium salt and (3) at least one acid, salt, base, and/or amino acid.
Examples of at least one ammonium salt in (2) include ammonium chloride, ammonium borate, ammonium sulfate, ammonium formate, ammonium acetate, ammonium citrate, ammonium tartrate, ammonium succinate, and ammonium phosphate and, one embodiment thereof is ammonium sulfate, ammonium citrate, and ammonium tartrate.
As for the acid, salt, base, and/or amino acid in (3), examples of the acid include ascorbic acid, benzoic acid, succinic acid, citric acid, glutamic acid, phosphoric acid, acetic acid, propionic acid, tartaric acid, carbonic acid, lactic acid, boric acid, maleic acid, fumaric acid, malic acid, adipic acid, hydrochloric acid, and sulfuric acid; examples of the salt include sodium salts of the aforementioned acids, potassium salts of the aforementioned acids, and ammonium salts of the aforementioned acids; examples of the base include trishydroxymethylaminomethane, ammonia, sodium hydroxide, and potassium hydroxide; and examples of the amino acid include arginine, histidine, and glycine.
In one embodiment of the liposomal composition according to the present disclosure, the acid, salt, base, and/or amino acid of (3) in the liposomal inner phase is hydrochloric acid, acetic acid, lactic acid, tartaric acid, succinic acid, citric acid, and phosphoric acid, sodium salts of the aforementioned acids, and sodium hydroxide and ammonia, and, in a particular embodiment, the acid, salt, base, and/or amino acid in (3) is acetic acid, lactic acid, tartaric acid, citric acid, and phosphoric acid, sodium salts of the aforementioned acids, and sodium hydroxide and ammonia.
An example of each component of the liposomal composition is set forth in Table 1. In another specific example, 96 mg/mL sucrose may be used, instead of 9 mg/mL sodium chloride, as an osmotic agent (liposomal outer phase).

[Table 1]

| Component | Concentration | Purpose of inclusion |
|---|---|---|
| Eribulin mesylate | 0.2 mg/mL | Drug |
| HSPC[1] | 7.1 mg/mL | Lipid membrane component |
| Cholesterol | 2.3 mg/mL | Lipid membrane component |
| MPEG2000-DSPE[2] | 2.7 mg/mL | Lipid membrane component |
| Ammonium sulfate | 100 mM | Liposomal inner phase component |
| Citric acid monohydrate | 30 mM | Liposomal inner phase component |
| Sodium chloride | 9 mg/mL | Liposomal outer phase component |
| L-histidine | 1.6 mg/mL | Liposomal outer phase component |
| Sodium hydroxide/hydrochloric acid | q.s. | pH adjuster |
| [1] Hydrogenated soy phosphatidylcholine<br>[2] N-{carbonyl-methoxy polyethylene glycol-2000}-1,2-distearoyl-sn-glycero-3-phosphoethanolamine (MPEG2000-distearoylphosphatidylethanolamine) | | |

[Table 2]

| Component | Concentration | Purpose of inclusion |
|---|---|---|
| Eribulin mesylate | 0.20 mg/mL | Drug |
| HSPC[1] | 7.10 mg/mL | Lipid membrane component |
| Cholesterol | 2.32 mg/mL | Lipid membrane component |
| MPEG2000-DSPE[2] | 2.69 mg/mL | Lipid membrane component |
| Ammonium sulfate | 100 mM | Liposomal inner phase component |
| Citric acid monohydrate | 30 mM | Liposomal inner phase component |
| Sucrose | 96 mg/mL | Liposomal outer phase component |
| L-histidine | 1.55 mg/mL | Liposomal outer phase component |
| Sodium hydroxide/hydrochloric acid | q.s. | pH adjuster |
| [1] Hydrogenated soy phosphatidylcholine<br>[2] N-{carbonyl-methoxy polyethylene glycol-2000}-1,2-distearoyl-sn-glycero-3-phosphoethanolamine (MPEG2000-distearoylphosphatidylethanolamine) | | |

The liposomal composition according to the present disclosure may be administered by injection (intravenous injection, intraarterial injection, topical injection), orally, nasally, transdermally, transpulmonarily, ophthalmically, and the like, and examples thereof include injection such as intravenous injection, subcutaneous injection, intradermal injection, intraarterial injection, as well as topical injection to a target cell and organ. Examples of the dosage form of the liposomal composition for oral administration include tablets, powders, granules, syrups, capsules, and oral solutions. Examples of the dosage form of the liposomal composition for parenteral administration include an injection, drip infusion, ophthalmic liquid, ointment, suppository, suspension, cataplasm, lotion, aerosol, and plaster, and one embodiment thereof is the injection or drip infusion. The liposomal composition according to the present disclosure may be formulated by a method, for example, described in Japanese Pharmacopoeia (JP) 17th edition, United States Pharmacopoeia (USP), or European pharmacopoeia (EP).

If the liposomal composition is a liquid formulation, it may be used as it is. If using the liposomal composition as a medicine, for example, a physician or a patient may inject a solvent into a vial in which a solid formulation is encapsulated to prepare upon use. If a solid formulation obtained by freezing a liquid liposomal composition, it may be stored in a frozen state and thawed by leaving at room temperature or thawed rapidly with heating back into a liquid upon use to be used as a liquid formulation.

The dose upon administration of the liposomal composition alone vary markedly depending on the kind of target disease, or the age, sex, body weight, the severity of symptoms, or the like of the patient. The liposomal composition is administered, for example, at 0.1 to 10 mg/m$^2$ (body surface) in terms of Eribulin mesylate per day for an adult. In one embodiment, the liposomal composition is administered at a dose of 0.5 to 3 mg/m$^2$ (body surface) in terms of Eribulin mesylate once every 1 week, 2 weeks, or 3 weeks. In a particular embodiment, the liposomal composition is more preferably administered at a dose of 0.5 to 2 mg/m$^2$ (body surface) in terms of Eribulin mesylate once every 1 week, 2 weeks, or 3 weeks.

In another embodiment, the liposomal composition is preferably administered at a dose of approximately 1.5 mg/m$^2$ (body surface) in terms of Eribulin mesylate once every 1 week, 2 weeks, or 3 weeks.

More specifically, the liposomal composition is administered intravenously at 0.5 to 1.4 mg/m$^2$ or 0.5 to 3.0 mg/m$^2$ on day 1 of a 21-day cycle or administered intravenously at 0.5 to 1.5 mg/m$^2$ or 0.5 to 3.0 mg/m$^2$ on day 1 and day 15 of a 28-day cycle in terms of Eribulin mesylate.

Eribulin or the like contained in the liposomal composition may be administered once a day or in several divided daily doses.

The liposomal composition may be a liposomal composition comprising, for example, 0.01 to 300 mg/mL of Eribulin or the like in the liposomal inner phase.

The liposomal composition is formulated, for example, as an injection comprising 0.20 mg/mL Eribulin mesylate (0.18 mg/mL Eribulin) incorporated in a liposome having a lipid membrane consisting of HSPC, cholesterol, and MPEG2000-DSPE. Such an injection may comprise sucrose or sodium chloride as an isotonizing agent, ammonium sulfate, citric acid, and L-histidine, and sodium hydroxide and hydrochloric acid to adjust pH. The injection is directly administered to a patient or diluted with physiological saline to be at the concentration range of 0.0035 mg/mL or higher and lower than 0.2 mg/mL before administration to a patient.

The PD-1 antagonist in the present disclosure may comprise any compound or biological molecule that blocks

the binding of PD-L1 expressed in cancer cells to PD-1 expressed in immune cells (T cells, B cells, or natural killer T (NKT) cells), or further blocks the binding of PD-L2 expressed in cancer cells to PD-1 expressed in immune cells. The PD-1 antagonist blocks the binding of human PD-L1 to human PD-1 and, in one embodiment, blocks the binding of both human PD-L1 and PD-L2 to human PD-1. The amino acid sequence of human PD-1 can be found in NCBI Locus No.: NP_005009. The amino acid sequences of human PD-L1 and PD-L2 can be found in NCBI Locus No: NP_054862 and NP_079515, respectively.

The PD-1 antagonist useful in the present disclosure may comprise a monoclonal antibody (mAb) or an antigen-binding fragment thereof that specifically binds to PD-1 or PD-L1 or specifically binds to human PD-1 or human PD-L1. The mAb may be a human antibody, humanized antibody, or chimeric antibody, and may comprise a human constant region. The human constant region is selected from the group consisting of IgG1, IgG2, IgG3, and IgG4 constant regions and, in one embodiment, the human constant region is an IgG1 or IgG4 constant region. The antigen-binding fragment may be selected from the group consisting of Fab, Fab'-SH, $F(ab')_2$, scFv, and Fv fragment.

An example of useful PD-1 antagonists is an anti-PD-1 antibody, which is, in one embodiment, an anti-human PD-1 antibody and, in a particular embodiment, an anti-human PD-1 monoclonal antibody (anti-human PD-1 mAb). Examples of the human PD-1-binding mAb binding are described in U.S. Patent No. 7488802, U.S. Patent No. 7521051, U.S. Patent No. 8008449, U.S. Patent No. 8354509, U.S. Patent No. 8168757, WO 2004/004771, WO 2004/072286, WO 2004/056875, and US Patent Application Publication No. 2011/0271358. Anti-human PD-1 monoclonal antibodies to be useful as the PD-1 antagonist according to the present disclosure include Nivolumab, Pembrolizumab, Cemiplimab, Sintilimab, and Toripalimab. Anti-PD-1 antibodies to be useful as the PD-1 antagonist according to the present disclosure further include Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

Another example of useful PD-1 antagonists is an anti-PD-L1 antibody, which is, in one embodiment, an anti-human PD-L1 antibody and, in a particular embodiment, an anti-human PD-L1 monoclonal antibody (anti-human PD-L1 mAb). Anti-PD-L1 antibodies to be useful as the PD-1 antagonist according to the present disclosure include Atezolizumab, Avelumab, Durvalumab, Manelimab, Pacmilimab, Envafolimab, Cosibelimab, BMS-936559, STI-1014, KN035, LY3300054, HLX20, SHR-1316, CS1001, MSB2311, BGB-A333 and KL-A16.

The PD-1 antagonist according to the present disclosure may be administered by injection (intravenous injection, intraarterial injection, topical injection), orally, nasally, transdermally, transpulmonarily, ophthalmically, and the like, and examples thereof include injection such as intravenous injection, subcutaneous injection, intradermal injection, intraarterial injection, as well as topical injection to target cells and organ. Examples of the dosage form of the PD-1 antagonist for oral administration include a tablet, powder, granule, syrup, capsule, and oral solution. Examples of the dosage form of the PD-1 antagonist for parenteral administration include an injection, drip infusion, ophthalmic liquid, ointment, suppository, suspension, cataplasm, lotion, aerosol, and plaster, and one embodiment thereof is the injection or drip infusion. The PD-1 antagonist according to the present disclosure may be formulated by a method, for example, described in Japanese Pharmacopoeia (JP) 17th edition, United States Pharmacopoeia (USP), or European pharmacopoeia (EP).

If the PD-1 antagonist according to the present disclosure is an anti-PD-1 antibody, the anti-PD-1 antibody may be provided as a liquid preparation or prepared by rehydrating its freeze-drying powder with sterile water for injection before use.

Upon administration of an anti-human PD-1 mAb alone as the PD-1 antagonist to a patient, the dose thereof varies markedly depending on the kind of target disease, or the age, sex, body weight, the severity of symptoms, or the like of the patient. The anti-human PD-1 mAb is administered, for example, at a dose of 1, 2, 3, 5, or 10 mg/kg at intervals of approximately 14 days (± 2 days), approximately 21 days (± 2 days), or approximately 30 days (± 2 days).

If Pembrolizumab is administered as the PD-1 antagonist, it is administered intravenously, for example, at a dose selected from the group consisting of 1 mg/kg Q2W, 2 mg/kg Q2W, 3 mg/kg Q2W, 5 mg/kg Q2W, 10 mg of Q2W, 1 mg/kg Q3W, 2 mg/kg Q3W, 3 mg/kg Q3W, 5 mg/kg Q3W, and 10 mg/kg Q3W. In another embodiment, Pembrolizumab is administered intravenously at a dose of 200 mg Q3W or 400 mg Q6W. Pembrolizumab is administered as a liquid medicine comprising, for example, 25 mg/ml Pembrolizumab, 7% (w/v) sucrose, and 0.02% (w/v) polysorbate 80 in a 10 mM histidine buffer, pH 5.5, and the medicine at a selected dose is administered by intravenous drip over a period of approximately 30 minutes.

If Nivolumab is administered as the PD-1 antagonist, it is administered intravenously, for example, at a dose selected from the group consisting of 1 mg/kg Q2W, 2 mg/kg Q2W, 3 mg/kg Q2W, 4 mg/kg Q2W, 5 mg/kg Q2W, 6 mg/kg Q2W, 1 mg/kg Q3W, 2 mg/kg Q3W, 3 mg/kg Q3W, 4 mg/kg Q3W, 5 mg/kg Q3W, 6 mg/kg Q3W, 8 mg/kg Q3W and 10 mg/kg Q3W. In another embodiment, Nivolumab is administered intravenously at a dose of 240 mg Q2W, 360 mg Q3W or 480 mg Q4W. For example, Nivolumab is administered by intravenous drip over a period of approximately 30 minutes or longer as a liquid medicine further comprising D-mannitol, sodium citrate hydrate, sodium chloride, diethylenetriaminepentaacetic acid, polysorbate 80 and a pH-adjusting agent.

If Cemiplimab is administered as the PD-1 antagonist, it is administered intravenously, for example, at a dose of 350 mg Q3W.

The doses of the liposomal composition and the PD-1 antagonist in the combined administration of the present disclosure may usually be set at doses lower than the doses when they are administered alone, respectively. Specific doses, administration routes, administration frequencies, administration cycles and the like are determined as appropriate in consideration of the kind of target disease, or the age, sex, body weight the severity of symptoms, or the like of the patient.

The modes of administration of the liposomal composition and the PD-1 antagonist in the present disclosure are not particularly limited, as long as the liposomal composition and the PD-1 antagonist are administered in combination when administered. For example, the liposomal composition and the PD-1 antagonist are administered to a patient simultaneously, separately, continuously, or at a time interval. Here, "simultaneously" means that each component is administered in the same period of time or strictly simultaneously, or via the same administration route. It also means that each component is administered without substantial interval between the both so as to produce an additive effect, preferably a synergistic effect. "Separately" means that each component is administered at different dose intervals or frequencies, or via different administration routes. "Continuously" means that each component is administered via the same or different administration routes in any order within a certain period of time. "At a time interval" means that each component is administered via the same or different administration routes, with each component being administered at a time interval. If the PD-1 antagonist is administered in a period of 1 cycle of the administration of the liposomal composition or a period in which the cycle is repeated, it is considered that both are administered in combination.

The mode of the combination is not particularly limited, and a person skilled in the art can combine the liposomal composition and the PD-1 antagonist in various ways.

In the case of combining a liposomal composition comprising Eribulin mesylate and Nivolumab, for example, Eribulin mesylate is intravenously administered at 1.7 to 2.1 mg/$m^2$ (body surface area) and Nivolumab is intravenously administered at 360 mg on day 1 of a 21-day cycle, or Eribulin mesylate is intravenously administered at 1.1 to 1.4 mg / $m^2$ (body surface area) and Nivolumab is intravenously administered at 240 mg on days 1 and 15 of a 28-day cycle.

A tumor to be treated in the present disclosure is breast cancer, gastric cancer, esophageal cancer, small cell lung cancer, colorectal cancer, renal cancer, thymic carcinoma or intrahepatic cholangiocarcinoma.

A patient to be treated in the present disclosure is, for example, a patient whose disease has progressed during or after a prior chemotherapy. Examples of the chemotherapy include a platinum-based drug, a fluorinated pyrimidine-based drug, a taxane-based drug, and combinations thereof.

A patient to be treated in the present disclosure is, for example, a patient with advanced, unresectable, or recurrent solid tumors without standard therapy or another effective therapy (a patient receiving Nivolumab monotherapy as standard therapy are eligible). In another embodiment, the patient is a patient with unresectable gastric cancer, esophageal cancer or small cell lung cancer, who has had a definitive diagnosis and whose disease progresses at the physician's discretion during or after a primary chemotherapy (or a secondary chemotherapy for gastric cancer) and who has not received other systemic chemotherapies for advanced or recurrent cancer thereof, wherein the patient is also a patient with gastric cancer administered with a combination chemotherapy comprising a platinum-based drug and a fluorinated pyrimidine-based drug as a primary chemotherapy and a chemotherapy comprising a taxane-based drug as a secondary chemotherapy, a patient with esophageal cancer administered with a combination chemotherapy comprising a platinum-based drug and a fluorinated pyrimidine-based drug (but not comprising a taxane-based drug) as a primary chemotherapy, or a patient with small cell lung cancer administered with a combination chemotherapy comprising a platinum-based drug as a primary chemotherapy. More specifically, the patient is a patient with intrahepatic cholangiocarcinoma administered with a combination therapy of Gemcitabine and Cisplatin as a primary therapy and a combination therapy of S-1 and Resminostat as a secondary therapy for a prior treatment, or a patient with thymic carcinoma administered with a combination therapy of Carboplatin and Paclitaxel as a primary therapy, S-1 as a secondary therapy and Gemcitabine as a tertiary therapy for a prior treatment.

## EXAMPLES

Liposomal compositions comprising Eribulin mesylate were prepared with the components set forth in Table 1 or Table 2 in accordance with the following methods and used in the following Examples.

Method of preparing the liposomal composition in Table 1

<Preparation of aqueous solution for liposomal inner phase>

Ammonium sulfate and citric acid monohydrate were dissolved and diluted with pure water to prepare an aqueous solution of 200 mM ammonium sulfate / 60 mM citric acid. The aqueous solution of 200 mM ammonium sulfate / 60

mM citric acid was adjusted to pH 5.5 with an aqueous ammonium solution and then diluted with pure water to obtain an aqueous solution of 100 mM ammonium sulfate / 30 mM citric acid.

<Preparation of liposomal preparatory liquid>

Hydrogenated soy phosphatidylcholine, cholesterol, and MPEG2000-distearoylphosphatidylethanolamine were weighted in accordance with a weight ratio of 71:23:27, respectively. These were dissolved in chloroform, respectively, and these solutions were mixed. Chloroform was then evaporated under reduced pressure in a rotary evaporator to prepare a lipid film. To the obtained lipid film, the prepared aqueous solution for liposomal inner phase heated to approximately 80°C was added and the resulting mixture was stirred to prepare a liposomal preparatory liquid. Sizing was performed using an extruder (a product made by Lipex Biomembranes Inc.) heated to approximately 80°C to obtain a sized liposomal preparatory liquid.

<Preparation of liposomal dispersion liquid>

By eluting the obtained liposomal preparatory liquid through a Sephadex G-50 column with an aqueous solution of 0.9% sodium chloride / 10 mM histidine (pH=7.6), the liposomal outer phase was exchanged into an aqueous solution of 0.9% sodium chloride / 10 mM histidine. After exchanging the liposomal outer phase, the liquid was centrifuged at 400,000xg for 30 minutes. After the centrifugation, re-dispersion was performed and the liquid volume was adjusted with an aqueous solution of 0.9% sodium chloride / 10 mM histidine to obtain a liposomal dispersion liquid.

<Preparation of Eribulin mesylate solution>

Eribulin mesylate was dissolved in an aqueous solution of 0.9% sodium chloride / 10 mM histidine to obtain an Eribulin mesylate solution.

<Preparation of liposomal composition>

The liposomal dispersion liquid and the Eribulin mesylate solution were mixed in a glass container and incubated in a water bath at 60°C for 3 minutes to obtain a liposomal composition with a liposomal inner phase in which Eribulin mesylate was introduced. An aqueous solution of 0.9% sodium chloride / 10 mM histidine was added to the liposomal composition and filter sterilization was performed with a 0.22 μm polyvinylidene fluoride (PVDF) filter to obtain an Eribulin mesylate liposomal composition.

Method 1 of preparing the liposomal composition in Table 2

<Preparation of aqueous solution for liposomal inner phase>

Ammonium sulfate and citric acid monohydrate were dissolved in pure water. The solution was adjusted to pH 7.5 with an aqueous solution of 1 mol/L sodium hydroxide, and then diluted with pure water to obtain an aqueous solution of 100 mM ammonium sulfate / 30 mM citric acid.

<Preparation of liposomal preparatory liquid>

To a lipid mixture comprising hydrogenated soy phosphatidylcholine, cholesterol and MPEG2000-distearoylphosphatidylethanolamine with a weight ratio of 710:232:269, were added the prepared aqueous solution for liposomal inner phase heated to approximately 80°C and the resulting mixture was stirred to prepare a liposomal preparatory liquid. Sizing was performed using an extruder (a product made by Lipex Biomembranes Inc.) heated to approximately 80°C to obtain a sized liposomal preparatory liquid.

<Preparation of liposomal dispersion liquid>

The liposomal outer phase was replaced with an aqueous solution of 96 mg/mL sucrose / 10 mM histidine by tangential flow filtration to obtain a liposomal dispersion liquid.

<Preparation of Eribulin mesylate solution>

Eribulin mesylate was dissolved in an aqueous solution of 96 mg/mL sucrose / 10 mM histidine to obtain an

Eribulin mesylate solution.

<Preparation of liposomal composition>

The liposomal dispersion liquid and the Eribulin mesylate solution were mixed and the resulting solution was adjusted to around pH 10 with hydrochloric acid or sodium hydroxide and incubated in a water bath at 60°C to introduce Eribulin mesylate into a liposomal inner phase. The solution was adjusted to pH 7.5 with hydrochloric acid or sodium hydroxide and an aqueous solution of 9.6 % sucrose / 10 mM histidine was added thereto to make Eribulin mesylate 0.2 mg/ml to obtain an Eribulin mesylate liposomal composition.

Method 2 of preparing the liposomal composition in Table 2

<Preparation of aqueous solution for liposomal inner phase>

105.68g of ammonium sulfate and 50.40g of citric acid monohydrate were dissolved in water for injection, and the resulting solution was adjusted to pH 7.5 with hydrochloric acid or sodium hydroxide to obtain an aqueous solution for liposomal inner phase.

<Preparation of liposomal preparatory liquid>

106.5 g of hydrogenated soy phosphatidylcholine, 34.8 g of cholesterol and 40.35 g of MPEG2000-distearoylphosphatidylethanolamine were dissolved in ethanol, and the solution was mixed with the aqueous solution for liposomal inner phase heated to approximately 70°C. To the resulting mixture was added water for injection to make a total weight of 8.26 kg. Sizing was performed to make a particle size of about 80 nm using an extruder (a product made by Lipex Biomembranes Inc.) heated to approximately 70°C to obtain a liposomal preparatory liquid.

<Preparation of liposomal dispersion liquid>

Pellicon 2 cassette ultrafiltration module Biomax (300 kDa) was used to replace the outer phase of the liposomal preparatory liquid with an aqueous solution of 9.6 % sucrose / 10 mM histidine (pH = 7.5) to obtain a liposomal dispersion liquid.

<Preparation of Eribulin mesylate solution>

Eribulin mesylate was dissolved in an aqueous solution of 9.6 % sucrose / 10 mM histidine to obtain an Eribulin mesylate solution.

<Preparation of liposomal composition>

The liposomal dispersion liquid and Eribulin mesylate solution were mixed and the resulting solution was adjusted to around pH 10 with hydrochloric acid or sodium hydroxide and incubated in a water bath at 60°C to introduce Eribulin mesylate into a liposomal inner phase. The solution was adjusted to pH 7.5 with hydrochloric acid or sodium hydroxide and an aqueous solution of 9.6 % sucrose / 10 mM histidine was added thereto to make 0.2 mg/ml of Eribulin mesylate to obtain an Eribulin mesylate liposomal composition.

[Example 1] Antitumor effect of combined administration of Eribulin mesylate at low dose (0.1 mg/kg) or Eribulin mesylate liposomal formulation at low dose (0.1 mg/kg) and anti-mouse PD-1 antibody in transplantation model of murine breast cancer 4T1 cell line with P glycoprotein knock-out (Pgp-KO 4T1)

A P glycoprotein-knockout cell line produced from murine breast cancer 4T1 cells (purchased from ATCC) was cultured using RPMI1640 medium (SIGMA) containing 10% of FBS (fetal bovine serum), 1 mM sodium pyruvate, and antibiotics, under conditions at 37°C in a 5% carbon dioxide gas incubator. The cells were collected using trypsin-EDTA when the cells reached to approximately 80% confluency. The medium described above (RPMI1640) was added to the collected cells, of which a suspension was prepared at $1.0 \times 10^7$ cells/mL, and 0.1 mL of the suspension was subcutaneously transplanted at the right body side into 6 mice (BALB/cAJcl, CLEA Japan, Inc.) per each group of the control group, the Eribulin mesylate alone administration group, the Eribulin mesylate liposomal formulation alone administration group, the anti-mouse PD-1 antibody (Bio X cell) alone administration group, the combined administration group of Eribulin mesylate and anti-mouse PD-1 antibody, and the combined administration group of Eribulin mesylate liposomal formulation and anti-mouse PD-1 antibody. From day 5 post-transplantation, Eribulin mesylate (0.1 mg/kg, once a week, twice in total, tail vein injection), the Eribulin mesylate liposomal formulation (0.1 mg/kg, once a week, twice in total, tail vein

injection), and the anti-mouse PD-1 antibody (200 μg/mouse, once a week, twice in total, tail vein injection) were administered, alone or in combination, to the alone administration groups or the anti-mouse PD-1 antibody combined administration groups, respectively. No drug was administered to the control group. The maximum tolerated dose of Eribulin mesylate liposomal formulation in mice is 2.5 mg/kg and the dose in this experiment was set very low at 0.1 mg/kg, which is 1/25 of the maximum tolerated dose.

The P-glycoprotein knockout cell line (Pgp-KO 4T1 cells) was prepared as follows. Single guide RNA (sgRNA) sequences were designed from the information of two types of P glycoprotein genes (mouse, Abcb1a and Abcblb) for knockout, and plasmid DNAs for animal cell expression (U6 promoter) into which the sgRNA for each gene was inserted were prepared (pRGEN_Mouse-Abcb1a_U6_SG_1 and pRGEN_Mouse-Abcb1b_U6_SG_1). In addition, Cas9 expressing plasmid DNA (CMV promoter) was prepared (pRGEN_CMV_Cas9). The prepared plasmid DNAs were purified by NucleoBond Xtra Midi EF and used for transfection. First, pRGEN_CMV_Cas9 and pRGEN_Mouse-Abcb1a_U6_SG_1 were used for gene editing for the first gene (Abcbla). Mouse breast cancer 4T1 cells (purchased from ATCC) were prepared in RPMI1640 medium (SIGMA) containing 10% FBS (fetal bovine serum) and antibiotics under conditions at 37°C in a 5% carbon dioxide gas incubator. After culturing, the cells were dissociated and collected using Trypsin-EDTA, and the above plasmid DNAs were transfected by a conventional method using electroporation (neopagan) under conditions of multiple mixing ratios. The cells for transfection were seeded on a 6-well microplate, cultured for 3 days, and then a portion of the cells was collected to determine whether or not genome editing was possible. Using the genomic DNA extracted from the collected cells by a conventional method, cleavage of the genomic DNA was confirmed using T7 Endonuclease I (T7E1) Assay. Cloning was performed from cells under mixing ratio in which more genomic DNA was cleaved in the T7E1 Assay. The cloned cells were appropriately subjected for enlarged culture, and cryopreserved, at the same time, the gene sequence was determined by sequence analysis to confirm the mutation of the target gene. One clone cell line was selected from multiple cloned cells in which mutation was confirmed, and gene editing (using pRGEN_CMV_Cas9 and pRGEN_Mouse-Abcb1b_U6_SG_1) for the second gene (Abcb1b) was performed in the same manner as described above. Finally, a 4T1 cell line (Pgp-KO 4T1 cells) was established in which two types of P-glycoprotein genes (mouse, Abcbla, and Abcb1b) were knocked out.

On day 3, day 7, day 10, day 13, day 16, day 20, day 23, day 27, day 30, and day 34 after administration, with the starting date of administration being day 0, the longest diameter and the short axis of the tumor grown in each mouse were measured with a digimatic caliper (a product made by Mitutoyo Corporation).

The tumor volume was calculated in accordance with the following formula.

$$\text{Tumor volume (mm}^3\text{) = longest diameter (mm) x short axis}^2\text{ (mm}^2\text{)/2}$$

The results of measurement of the tumor volume in each group are illustrated as mean and standard deviation (SD) in FIG. 1. As statistical analysis, repeated measures analysis of variance followed by Dunnett's multiple comparison was conducted in comparison with the control group for tumor volumes on all measurement days in all groups (*: $p < 0.05$, ***: $p < 0.001$). The statistical comparison between two groups of the combined administration group of Eribulin mesylate and anti-mouse PD-1 antibody and the combined administration group of Eribulin mesylate liposomal formulation and anti-mouse PD-1 antibody was conducted by repeated measures analysis of variance (#: $p < 0.05$).

As a result, the combined administration of Eribulin mesylate liposomal formulation at low dose (0.1 mg/kg) and anti-mouse PD-1 antibody exhibited a remarkable antitumor effect in comparison with the control group in the Pgp-KO 4T1 tumor transplantation model. * and *** in FIG. 1 indicate that the combined administration of Eribulin mesylate liposomal formulation and anti-mouse PD-1 antibody statistically significantly inhibited tumor growth in comparison with the control group. # indicates that the combined administration of Eribulin mesylate liposomal formulation and anti-mouse PD-1 antibody statistically significantly inhibited tumor growth in comparison with the combined administration of Eribulin mesylate and anti-mouse PD-1 antibody. In contrast, no antitumor effect was observed in the Eribulin mesylate (0.1 mg/kg) alone administration and the Eribulin mesylate liposomal formulation (0.1 mg/kg) alone administration, which are at low doses, and the anti-mouse PD-1 antibody alone administration, and even in the combined administration of Eribulin mesylate (0.1 mg/kg) and anti-mouse PD-1 antibody.

The result of comparison among the groups for the time until the tumor volume exceeds 5 times that on the starting date of administration (Tx5) in the experiment is shown in FIG. 2 and the median of Tx5 in each group and the respective percentages (%) thereof to the control group are set forth in Table 3. For statistical analysis, the Log-rank test was conducted in comparison with the control group to calculate the Bonferroni-corrected p-value (*: $p < 0.05$).

As a result, the combined administration of Eribulin mesylate liposomal formulation (0.1 mg/kg) and anti-mouse PD-1 antibody exhibited the effect of extending (243%) the time of suppressing tumor growth (Tx5) in Pgp-KO 4T1 tumor transplantation model. In contrast, no extension effect was observed in the Eribulin mesylate (0.1 mg/kg) alone administration and the Eribulin mesylate liposomal formulation (0.1 mg/kg) alone administration, which are at low doses, and the anti-mouse PD-1 antibody alone administration, and even in the combined administration of Eribulin mesylate and

anti-mouse PD-1 antibody. * in Table 3 indicates that the combined administration of Eribulin mesylate liposomal formulation (0.1 mg/kg) and anti-mouse PD-1 antibody statistically significantly extended the time of tumor growth suppression in comparison with the control group.

[Table 3]

| Effect of the combined administration of Eribulin mesylate liposomal formulation (0.1 mg/kg) and anti-mouse PD-1 antibody on Tx5 | | |
|---|---|---|
| Group | Tx5 (days) | Percentage to control |
| Control | 10.5 | 100% |
| Eribulin mesylate Alone | 12.0 | 114% |
| Eribulin mesylate liposomal formulation Alone | 11.5 | 110% |
| Anti-mouse PD-1 antibody Alone | 14.5 | 138% |
| Eribulin mesylate + Anti-mouse PD-1 antibody Combined administration | 14.5 | 138% |
| Eribulin mesylate liposomal formulation + Anti-mouse PD-1 antibody Combined administration | 25.5* | 243% |

[Example 2] Antitumor effect of combined administration of Eribulin mesylate at low dose (0.3 mg/kg) or Eribulin mesylate liposomal formulation at low dose (0.3 mg/kg) and anti-mouse PD-1 antibody in Pgp-KO 4T1 cell line transplantation model

Pgp-KO 4T1 cells were cultured with the RPMI1640 medium containing 10% FBS, 1 mM sodium pyruvate, and antibiotics under conditions at 37°C in a 5% carbon dioxide gas incubator. The cells were collected using trypsin-EDTA when the cells reached to approximately 80% confluency. The medium described above was added to the collected cells to prepare a suspension at $1.0x10^7$ cells/mL. 0.1 mL of the cell suspension was subcutaneously transplanted at the right body side into 6 mice (BALB/cAJcl, CLEA Japan, Inc.) per each group of the control group, the Eribulin mesylate liposomal formulation alone administration group, the anti-mouse PD-1 antibody (Bio X cell) alone administration group, and the combined administration of Eribulin mesylate liposomal formulation and anti-mouse PD-1 antibody. From day 4 post-transplantation, the Eribulin mesylate liposomal formulation (0.3 mg/kg, once a week, twice in total, tail vein injection) and the anti-mouse PD-1 antibody (200 μg/mouse, once a week, twice in total, tail vein injection) were administered, alone or in combination, to the alone administration groups or the combined administration group, respectively. No drug was administered to the control group.

On day 3, day 7, day 9, day 13, day 17, day 20, day 24, day 27, day 31, day 34, day 38, day 41, day 44, day 48, and day 51 after administration, with the starting date of administration being day 0, the longest diameter and the short axis of the tumor grown in each mouse were measured with a digimatic caliper (a product made by Mitutoyo Corporation).

The tumor volume was calculated in accordance with the following formula.

$$\text{Tumor volume } (mm^3) = \text{longest diameter } (mm) \text{ x short axis}^2 \text{ } (mm^2)/2$$

The mean and standard deviation (SD) of the results of measurement of the tumor volume in each group are illustrated in FIG. 3 and the frequencies of mice with tumor disappearance are set forth in Table 4. For statistical analysis, the statistical comparison between two groups of the Eribulin mesylate liposomal formulation alone administration group or the anti-mouse PD-1 antibody alone administration group and the combined administration group of Eribulin mesylate liposomal formulation and anti-mouse PD-1 antibody was conducted by repeated measures analysis of variance (†, #: $p < 0.05$).

As a result, in the Pgp-KO 4T1 tumor transplantation model, the combined administration of Eribulin mesylate liposomal formulation at low dose (0.3 mg/kg) and anti-mouse PD-1 antibody exhibited excellent antitumor effect in comparison with the Eribulin mesylate liposomal formulation alone administration group or anti-mouse PD-1 antibody alone administration group. In FIG. 3, (†) indicates that the combined administration of Eribulin mesylate liposomal formulation and anti-mouse PD-1 antibody statistically significantly inhibited tumor growth in comparison with the Eribulin mesylate liposomal formulation alone administration and (#) indicates that the combined administration of Eribulin mesylate liposomal formulation and anti-mouse PD-1 antibody statistically significantly inhibited tumor growth in comparison with the anti-mouse PD-1 antibody alone administration. The tumor disappearance was observed in the combined administration group of Eribulin mesylate liposomal formulation (0.3 mg/kg) and anti-mouse PD-1 antibody at a frequency higher than other groups.

[Table 4]

| Frequency of appearance of mice with tumor disappearance in each group | |
|---|---|
| Group | Frequency (%) of mice with tumor disappearance |
| Control | 0/6 (0%) |
| Eribulin mesylate liposomal formulation Alone | 1/6 (17%) |
| Anti-mouse PD-1 antibody Alone | 0/6 (0%) |
| Eribulin mesylate liposomal formulation + Anti-mouse PD-1 antibody Combined administration | 4/6 (67%) |

The result of comparison of the groups for the time until the tumor volume exceeds 5 times that on the starting date of administration (Tx5) in the experiment is shown in FIG. 4 and the median of Tx5 in each group and the percentage (%) thereof to the control group are set forth in Table 5. For statistical analysis, the Log-rank test between two groups of the combined administration group of Eribulin mesylate liposomal formulation and anti-mouse PD-1 antibody to the anti-mouse PD-1 antibody alone administration group was conducted (#: $p < 0.05$).

As a result, in the Pgp-KO 4T1 tumor transplantation model, the combined administration of Eribulin mesylate liposomal formulation (0.3 mg/kg) and the anti-mouse PD-1 antibody exhibited the effect of extending suppression time of tumor growth (Tx5) in comparison with the control group, the Eribulin mesylate liposomal formulation alone administration group, and the anti-mouse PD-1 antibody alone administration group. # in Table 5 indicates that the combined administration of Eribulin mesylate liposomal formulation and anti-mouse PD-1 antibody statistically significantly extended suppression time of tumor growth in comparison with the anti-mouse PD-1 antibody alone administration.

[Table 5]

| Effect of combined administration of Eribulin mesylate liposomal formulation (0.3 mg/kg) and anti-mouse PD-1 antibody to Tx5 | | |
|---|---|---|
| Group | Tx5 (days) | Ratio to control |
| Control | 11.5 | 100% |
| Eribulin mesylate liposomal formulation Alone | 33.0 | 287% |
| Anti-mouse PD-1 antibody Alone | 18.0 | 157% |
| Eribulin mesylate liposomal formulation + Anti-mouse PD-1 antibody Combined administration | >51.0# | >443% |

[Example 3] Antitumor effect of combined administration of Eribulin mesylate at low dose (0.3 mg/kg) or Eribulin mesylate liposomal formulation at low dose (0.3 mg/kg) and anti-mouse PD-1 antibody in transplantation model of murine colorectal cancer CT-26 cell line with P glycoprotein knock-out (Pgp-KO CT-26)

From mouse colorectal cancer CT-26 cells (purchased from ATCC), a P glycoprotein knockout cell line (Pgp-KO CT-26) was produced in accordance with the same materials and procedures used in the production of Pgp-KO 4T1 cells. Pgp-KO CT-26 cells were cultured using RPMI1640 medium (FUJIFILM Wako Pure Chemical Corporation) containing 10% of FBS (fetal bovine serum), and antibiotics, under conditions at 37°C in a 5% carbon dioxide gas incubator. The cells were collected using trypsin-EDTA when the cells reached to approximately 80% confluency. HBSS medium was added to the collected cells, of which a suspension was prepared at $2.0x10^7$ cells/mL, and 0.1 mL of the suspension was subcutaneously transplanted at the right body side into 7 mice (BALB/cAJcl, CLEA Japan, Inc.) per each group of the control group, the Eribulin mesylate alone administration group, the Eribulin mesylate liposomal formulation alone administration group, the anti-mouse PD-1 antibody (Bio X cell) alone administration group, the combined administration group of Eribulin mesylate and anti-mouse PD-1 antibody, and the combined administration group of Eribulin mesylate liposomal formulation and anti-mouse PD-1 antibody. From day 15 post-transplantation, Eribulin mesylate (0.3 mg/kg, once a week, three times in total, tail vein injection), Eribulin mesylate liposomal formulation (0.3 mg/kg, once a week, three times in total, tail vein injection), and the anti-mouse PD-1 antibody (200 μg/mouse, once a week, three times in total, tail vein injection) were administered, alone or in combination, to the alone administration groups or anti-mouse PD-1 antibody combined administration groups, respectively. No drug was administered to the control group.

With the starting date of administration being day 0, the longest diameter and the short axis of the tumor grown in each mouse were measured with time to Day 18 with a digimatic caliper (a product made by Mitutoyo Corporation).

The tumor volume was calculated in accordance with the following formula.

$$\text{Tumor volume (mm}^3\text{)} = \text{longest diameter (mm) x short axis}^2\text{ (mm}^2\text{)/2}$$

The results of measurement of the tumor volume in each group are illustrated as mean and standard deviation (SD) in Table 6. As statistical analysis, repeated measures analysis of variance followed by Dunnett's multiple comparison was conducted in comparison with the control group for tumor volumes in all groups (*: $p < 0.05$,).

As a result, the combined administration of Eribulin mesylate liposomal formulation at low dose (0.3 mg/kg) and anti-mouse PD-1 antibody exhibited a remarkable antitumor effect in comparison with the control group in the Pgp-KO CT-26 tumor transplantation model. * in Table 6 indicate that the combined administration of Eribulin mesylate liposomal formulation and the anti-mouse PD-1 antibody statistically significantly inhibited tumor growth in comparison with the control group. In contrast, no antitumor effect at low doses with the Eribulin mesylate (0.3 mg/kg) alone administration, the Eribulin mesylate liposomal formulation (0.3 mg/kg) alone administration, the anti-mouse PD-1 antibody alone administration, and even in the combined administration of Eribulin mesylate (0.3 mg/kg) and anti-mouse PD-1 antibody was observed with a significant difference in comparison with the control group.

[Table 6]

| Effect of the combined treatment of Eribulin mesylate liposomal formulation (0.3 mg/kg) and anti-mouse PD-1 antibody on tumor growth in Pgp-KO CT-26 tumor transplantation model | |
|---|---|
| Group | Tumor volume |
| Control | 1851 ± 517 ($mm^3$) |
| Eribulin mesylate Alone | 1139 ± 733 ($mm^3$) |
| Eribulin mesylate liposomal formulation Alone | 1012 ± 575 ($mm^3$) |
| Anti-mouse PD-1 antibody Alone | 1378 ± 632 ($mm^3$) |
| Eribulin mesylate + Anti-mouse PD-1 antibody Combined administration | 1170 ± 772 ($mm^3$) |
| Eribulin mesylate liposomal formulation + Anti-mouse PD-1 antibody Combined administration | 770 ± 593 ($mm^3$)* |

[Example 4] Antitumor effect of combined administration of Eribulin mesylate liposomal formulation at low dose (0.3 mg/kg) and anti-mouse PD-1 antibody in a mouse renal cancer RAG cell line transplantation model

RAG cells (purchased from ATCC) used in the transplant model were conditioned in mice (BALB/cAnNCrlCrlj, Charles River Laboratories, Japan) in advance. RAG cells were suspended in HBSS (Wako Pure Chemical Industries, Ltd.) at a concentration of $2x10^8$ cells/mL. To this suspension was added the same volume of Matrigel™ matrix (Nippon Becton Dickinson Co., Ltd.) and mixed well. The mixture was implanted into the subcutaneous region of the right flank of each mouse at 0.1 mL. On day 38 post-transplantation, the generated tumor was removed and cut into small pieces, and Tumor Dissociation Kit, Mouse (Miltenyi) was added thereto, and stirred using Gentle MACS (Miltenyi). After passing through a 70 μm cell strainer, the cells were collected by centrifugation, and cultured in an EMEM medium (Wako Pure Chemical Industries, Ltd.) containing 10% bovine serum under conditions at 37°C in a 5% carbon dioxide gas incubator and cryopreserved.

The cells obtained by the above treatment were cultured using EMEM medium containing 10% FBS and antibiotics. The cells were collected using trypsin-EDTA when the cells reached to approximately 80% confluency. HBSS was added to the collected cells to prepare a suspension at $2.5x10^7$ cells/mL. 0.1 mL of the cell suspension was subcutaneously transplanted at the right body side into 10 mice (BALB/cAnNCrlCrlj, Charles River Laboratories, Japan) per each group of the control group, the Eribulin mesylate liposomal formulation alone administration group, the anti-mouse PD-1 antibody (Bio X cell) alone administration group, and the combined administration group of Eribulin mesylate liposomal formulation and anti-mouse PD-1 antibody. From day 6 post-transplantation, the Eribulin mesylate liposomal formulation (0.3 mg/kg, once a week, three times in total, tail vein injection) and the anti-mouse PD-1 antibody (200 μg/mouse, once every 3 days, ten times in total, intraperitoneal administration) were administered alone or in combination to the alone administration groups or the combined administration group, respectively. No drug was administered to the control group.

On day 3, day 7, day 10, day 14, day 17, day 21, day 24, day 28, day 31, day 35, day 38 and day 42 after administration, with the starting date of administration being day 0, the longest diameter and the short axis of the tumor grown in each mouse were measured with a digimatic caliper (a product made by Mitutoyo Corporation).

The tumor volume was calculated in accordance with the following formula.

$$\text{Tumor volume (mm}^3\text{) = longest diameter (mm) x short axis}^2\text{ (mm}^2\text{)/2}$$

Table 7 shows the frequencies of mice with tumor shrinkage of 50% or more at best in each group as the response rate, and Table 8 shows the frequencies of mice with tumor disappearance. For statistical analysis, the comparison between the Eribulin mesylate liposomal formulation alone administration group, the anti-mouse PD-1 antibody alone administration group, or the combined administration group of Eribulin mesylate liposomal formulation and anti-mouse PD-1 antibody and control group was conducted by 2-sided Fisher's exact test (#: $p < 0.05$).

As a result, in the RAG tumor transplantation model, the combined administration of Eribulin mesylate liposomal formulation at low dose (0.3 mg/kg) and anti-mouse PD-1 antibody exhibited high response in comparison with the Eribulin mesylate liposomal formulation alone administration group or the anti-mouse PD-1 antibody alone administration group. In Table 7, (*) indicates that the combined administration of Eribulin mesylate liposomal formulation and anti-mouse PD-1 antibody statistically significantly responded. On the other hand, no significant response was observed with the Eribulin mesylate liposomal formulation (0.3 mg / kg) alone administration or the anti-mouse PD-1 antibody alone administration. The tumor disappearance was observed in the combined administration group of Eribulin mesylate liposomal formulation (0.3 mg/kg) and anti-mouse PD-1 antibody at a frequency higher than other groups.

[Table 7]

| Response rate in each group | |
|---|---|
| Group | Number of mice responded / Number of mice in each group (Response rate %) |
| Control | 0/10 (0%) |
| Eribulin mesylate liposomal formulation Alone | 4/10 (40%) |
| Anti-mouse PD-1 antibody Alone | 2/10 (20%) |
| Eribulin mesylate liposomal formulation + Anti-mouse PD-1 antibody Combined administration | 5/10 (50%) |

[Table 8]

| Frequency of appearance of mice with tumor disappearance in each group | |
|---|---|
| Group | Frequency (%) of mice with tumor disappearance |
| Control | 0/10 (0%) |
| Eribulin mesylate liposomal formulation Alone | 2/10 (20%) |
| Anti-mouse PD-1 antibody Alone | 1/10 (10%) |
| Eribulin mesylate liposomal formulation + Anti-mouse PD-1 antibody Combined administration | 4/10 (40%) |

Example 5

Open-label, Phase 1b/2 study of combination of Eribulin mesylate liposomal formulation and Nivolumab in patients with solid tumors

The study is multicenter, single-group, and Open-label study. The Phase 1b part of the study is for patients with solid tumors without standard therapy or another effective therapy, and the Phase 2 part is for patients with esophageal cancer having progressed during or after a primary chemotherapy (a combination chemotherapy comprising a platinum-based drug and a fluorinated pyrimidine-based drug), patients with small cell lung cancer having progressed during or after a primary chemotherapy (a combination chemotherapy comprising a platinum-based drug), or patient with gastric cancer having progressed during or after a secondary chemotherapy (a combination chemotherapy comprising a platinum-based drug and a fluorinated pyrimidine-based drug as a primary chemotherapy and a chemotherapy comprising a taxane-based drug as a secondary chemotherapy.

The study consists of three periods: the pre-administration period, the administration period, and the follow-up period (only for Phase 2 part). The pre-administration period includes screening for checking the eligibility and baseline for checking the disease status and it was within 28 days. During the administration period, subjects received administration of Eribulin mesylate liposomal formulation and Nivolumab. Tumor assessment was performed every 6 weeks from the start of administration. The follow-up period (only for Phase 2 part) is initiated after discontinuation of Eribulin mesylate liposomal formulation and Nivolumab to investigate the survival and post-treatment of the subjects.

In the Phase 1b part, two administration methods: Schedule 1 and Schedule 2, were examined. In Schedule 1, Eribulin mesylate liposomal formulation and Nivolumab were administered intravenously on day 1 of 21-day cycle, and the dose of Nivolumab was 360 mg, and the dose of Eribulin mesylate liposomal formulation (as mesylate) was 1.7 or 2.1 mg / $m^2$. In Schedule 2, Eribulin mesylate liposomal formulation and Nivolumab were administered intravenously on day 1 and day 15 of 28-day cycle, and the dose of Nivolumab was 240 mg, and the dose of Eribulin mesylate liposomal formulation (as mesylate) was 1.1 or 1.4 mg / $m^2$.

In the phase 2 part, the recommended dosage determined in the phase 1b part is used.

The primary objective of the study is to evaluate the safety and tolerability of the combination of Eribulin mesylate liposomal formulation and Nivolumab to determine the recommended dosage for Phase 2 (Phase 1b part), and to evaluate the objective response rate in each cancer at the recommended dosage (Phase 2 part). The secondary objective of the study is to evaluate the safety, pharmacokinetics and progression-free survival of the combination of Eribulin mesylate liposomal formulation and Nivolumab.

Patients who meet all of the following criteria are eligible for the study. Inclusion criteria

(1) only for Phase 1b part:
Patients with advanced, unresectable or recurrent solid tumors without standard therapy or another effective therapy (patients receiving Nivolumab monotherapy as standard therapy are eligible)
(2) only for Phase 2 part:
Patient with unresectable gastric cancer, esophageal cancer or small cell lung cancer who have had a definitive diagnosis and whose disease progresses at the physician's discretion during or after a primary chemotherapy (or a secondary chemotherapy for gastric cancer) and who have not received other systemic chemotherapy for advanced or recurrent cancer
(3) only for Phase 2 part:

Patients who received the following chemotherapy as a prior chemotherapy Gastric cancer: a combination chemotherapy comprising a platinum-based drug and a fluorinated pyrimidine-based drug as a primary chemotherapy and a chemotherapy comprising a taxane-based drug as a secondary chemotherapy Esophageal cancer: a combination chemotherapy comprising a platinum-based drug and a fluorinated pyrimidine-based drug (but not comprising a taxane-based drug) as a primary chemotherapy
Small cell lung cancer: a combination chemotherapy comprising a platinum-based drug as a primary chemotherapy

(4) Patients with tumor lesions that can be biopsied and who consent to tumor biopsy before and after administration of the investigational drug (If biopsy before administration is not possible due to safety concerns, submission of preserved tumor specimens is permitted)
(5) Patients who are expected to survive for 12 weeks or longer
(6) Patients with ECOG-PS 0-1
(7) Japanese patient aged 20 or above at the time of consent
(8) Patients whose adverse events (excluding hair loss and Grade 2 peripheral neuropathy) due to a prior therapy for cancer have recovered to Grade 0-1 (renal / bone marrow / liver / pancreatic function must be recovered to meets inclusion criteria)
(9) Patients who have passed the following period from the end of a prior therapy to C1D1 (cycle 1 day 1)

a) Non-cytotoxic anti-cancer drug: 4 weeks (or 5 half-lives, whichever shorter) or longer
b) Cytotoxic anti-cancer drug and radiation therapy: 3 weeks or longer
c) Treatment with anti-cancer drug of antibody preparation: 4 weeks or longer
d) Investigative drug or medical device under investigation: 4 weeks or longer
e) Blood transfusion, platelet transfusion or G-CSF preparation: 2 weeks or longer
f) Live or attenuated vaccine: 4 weeks or longer

(10) Patients with appropriate renal function: serum creatinine ≤ 1.5 x upper limit of normal (ULN) (in the case of > 1.5 x ULN, patients having creatinine clearance by the Cockcroft-Gault method ≥ 40 mL / min are eligible)

(11) Patients with appropriate bone marrow function:

a) neutrophil count ≥ 2,000 / $mm^3$
b) platelet count ≥ 100,000 / $mm^3$
c) hemoglobin ≥ 8.5 g / dL

(12) Patients with appropriate liver function:

a) international normalized ratio (INR) as anticoagulation ability ≤ 1.5
b) total bilirubin ≤1.5 x ULN (for Gilbert's syndrome patient ≤3.0 x ULN)
c) alkaline phosphatase (ALP), alanine aminotransferase (ALT) and aspartate aminotransferase (AST) ≤3 x ULN (for patient having liver lesions ≤5 x ULN) (13) Patients with appropriate pancreatic function: amylase and lipase ≤1.5 x ULN (for patients having pancreatic lesions ≤3 x ULN) (14) only for Phase 2 part: Patients with measurable lesions in RECIST 1.1 (when lesions that have undergone radiation therapy or topical therapy are regarded as measurable lesions, the lesions must be aggravated) (15) Patients who can consent to documents and comply with the clinical trial protocol

Patients who meet any of the following criteria will be excluded from the study.

Exclusion criteria

(1) Patients with any of the following cardiac diseases

New York Heart Association (NYHA) class II or higher cardiac failure
Unstable ischemic heart disease (myocardial infarction within 6 months prior to C1D1, angina requiring at least 2 times a week of nitrovasodilator)
QTcF > 480 ms prolonged QT interval

(2) Patients with a history of hypersensitivity reactions to liposomal preparations
(3) Patients who underwent major surgery within 21 days from C1D1
(4) Patients with a history of Eribulin or Nivolumab
(5) Patients who experienced Grade 3 or higher immune-related adverse events or discontinued treatment with anti-PD-1 antibodies, anti-PD-L1 antibodies, anti-PD-L2 antibodies, anti-CD137 antibodies, anti-CTLA-4 antibodies, or other antibodies and drugs that target T-cell co-stimulation or checkpoints, or cancer vaccines
(6) Patients who are known to be intolerant to the investigational drug or any of its additives or antibody preparations
(7) Patients with known human immunodeficiency virus (HIV) positive or acquired immunodeficiency syndrome (AIDS)
(8) Patients with active hepatitis B or C (hepatitis B virus surface [HBs] antigen positive, HBs antigen negative and anti-HBs antibody-positive or anti-HBc antibody positive and HBV-DNA positive, or HCV-RNA positive)
(9) Patients with active infections who required systemic treatment within 14 days from C1D1
(10) Patients with membrane carcinomatosis
(11) Patients with brain or subdural metastasis or invasion. Excluded are Patients who have completed topical treatment and have discontinued corticosteroids 4 weeks before C1D1. Signs (e.g. on radiological examination) and symptoms should be stable for 4 weeks from C1D1.
(12) Patients with pulmonary lymphangitis and respiratory failure who require active treatment (including oxygen inhalation)
(13) Patients with active or known autoimmune diseases or suspected autoimmune diseases. Except:

Patients with vitiligo vulgaris, type I diabetes, recovered childhood asthma, or atopy
Patients with suspected autoimmune thyroid disorders who have normal current thyroid function or who can maintain normal thyroid function with hormone replacement therapy alone for residual hypothyroidism

(14) Patients with or having a history of clinically or image-diagnosed interstitial lung disease or pulmonary fibrosis (patients with radiation pneumonitis with confirmed stable fibrosis and no risk of recurrence are eligible)
(15) Patients having a history of organ transplantation requiring immunosuppressive drug administration
(16) Patients who require systemic administration of corticosteroids (equivalent to more than 10 mg / day of

prednisolone) or other immunosuppressive drugs within 14 days from C1D1. Patients using inhaled or topically administered corticosteroids (if less absorbed in the body) can be enrolled in the absence of active autoimmune disease.

(17) Only for Phase 2 part: Patients with active malignancies within 24 months from C1D1 (primary disease, fully treated non-invasive melanoma, basal cell carcinoma of skin / squamous cell carcinoma, non-invasive cervical cancer / bladder cancer and early gastric cancer / colorectal cancer are excluded)

(18) Patients with clinically significant disease / condition (e.g., heart, respiratory, digestive, renal disease) determined by the investigator or subinvestigator to affect subject safety or study evaluation

(19) For women, patients who are lactating or pregnant at screening or at baseline (patients tested positive for human chorionic gonadotropin [hCG] or beta subunit of human chorionic gonadotropin [β-hCG]). If the screening is negative but not within 72 hours of C1D1, the test should be repeated.

(20) In the case of fertile men and women of childbearing potential, patients who do not agree to use medically appropriate contraceptive methods[NOTE)] for the duration of the study and until 5 months (7 months for male subjects) after the administration of the investigational drug.

Note) Double contraception with two of the following: vasectomy, tubal ligation, use of condom*, contraceptive sponge**, contraceptive foam**, contraceptive gel**, contraceptive diaphragm*, or contraceptive ring*, or use of oral contraceptive* for 28 days or more before the start of administration of this investigational drug (whether or not approval or certification of drugs and medical devices in Japan [*: Yes, **: No])

The administration of Eribulin mesylate liposomal formulation and Nivolumab was decided to continue until disease progression, intolerable side effects occurrence, subject's request for discontinuation, consent withdrawal, or the sponsor's discontinuation of the study.

Interim results of Phase 1b part

Case 1

A case of intrahepatic cholangiocarcinoma (Schedule 2, a dose of Eribulin mesylate liposomal formulation is 1.1 mg / $m^2$)

To a patient who received a combination therapy of Gemcitabine and Cisplatin as a primary therapy, a combination therapy of S-1 and Resminostat as a secondary therapy, and an investigational drug (details unknown) as a tertiary therapy, for a prior treatment, were administered 1.1 mg / $m^2$ of Eribulin mesylate liposomal formulation and 240 mg of Nivolumab once every two weeks in Schedule 2. After administration, at Week 6/12/18/24/30/36, the sum of diameters of the target lesions from the baseline was reduced by 10.3 %, 14.7 %, 23.5 %, 40.8 %, 46.2 % and 51.4 % from the baseline, respectively, and it was considered as a partial response.

Case 2

A case of thymic carcinoma (Schedule 1, a dose of Eribulin mesylate liposomal formulation is 1.7 mg / $m^2$)

To a patient who received a combination therapy of Carboplatin and Paclitaxel as a primary therapy, S-1 as a secondary therapy, Gemcitabine as a tertiary therapy, an investigational drug (a combination of an unknown drug and a PD-L1 antibody) as a quaternary therapy and an investigational drug (details unknown) as a quinary therapy, for a prior treatment, 1.7 mg / $m^2$ of Eribulin mesylate liposomal formulation and 360 mg of Nivolumab were administered once every three weeks in Schedule 1. After administration, at Week 6/12/18, the sum of diameters of the target lesions was reduced by 17.8 %, 100 % and 100 % from the baseline, respectively, and it was considered as a partial response.

## Claims

1. A pharmaceutical composition for use in treating tumor, comprising a liposomal composition comprising Eribulin or a pharmaceutically acceptable salt thereof in the liposomal inner phase,

   wherein the pharmaceutical composition is administered in combination with a programmed cell death 1 protein (PD-1) antagonist, and

   wherein the tumor is breast cancer, gastric cancer, esophageal cancer, small cell lung cancer, colorectal cancer, renal cancer, thymic carcinoma or intrahepatic cholangiocarcinoma.

2. A pharmaceutical composition for use in treating tumor, comprising a programmed cell death 1 protein (PD-1)

antagonist,

wherein the pharmaceutical composition is administered in combination with a liposomal composition comprising Eribulin or a pharmaceutically acceptable salt thereof in the liposomal inner phase, and
wherein the tumor is breast cancer, gastric cancer, esophageal cancer, small cell lung cancer, colorectal cancer, renal cancer, thymic carcinoma or intrahepatic cholangiocarcinoma.

**3.** A liposomal composition comprising Eribulin or a pharmaceutically acceptable salt thereof in the liposomal inner phase for use in treating tumor,

wherein the liposomal composition is administered in combination with a PD-1 antagonist, and
wherein the tumor is breast cancer, gastric cancer, esophageal cancer, small cell lung cancer, colorectal cancer, renal cancer, thymic carcinoma or intrahepatic cholangiocarcinoma.

**4.** A PD-1 antagonist for use in treating tumor,

wherein the PD-1 antagonist is administered in combination with a liposomal composition comprising Eribulin or a pharmaceutically acceptable salt thereof in the liposomal inner phase, and
wherein the tumor is breast cancer, gastric cancer, esophageal cancer, small cell lung cancer, colorectal cancer, renal cancer, thymic carcinoma or intrahepatic cholangiocarcinoma.

**5.** The pharmaceutical composition for use, the liposomal composition for use, or the PD-1 antagonist for use according to any one of claims 1 to 4, wherein the liposomal composition comprising Eribulin or the pharmaceutically acceptable salt thereof in the liposomal inner phase and the PD-1 antagonist are administered simultaneously, separately, continuously, or at a time interval.

**6.** The pharmaceutical composition for use, the liposomal composition for use, or the PD-1 antagonist for use according to any one of claims 1 to 5, wherein Eribulin or the pharmaceutically acceptable salt thereof is Eribulin mesylate.

**7.** The pharmaceutical composition for use, the liposomal composition for use, or the PD-1 antagonist for use according to any one of claims 1 to 6, wherein the PD-1 antagonist is an anti-PD-1 antibody.

**8.** The pharmaceutical composition for use, the liposomal composition for use, or the PD-1 antagonist for use according to claim 7 wherein the anti-PD-1 antibody is selected from the group consisting of Nivolumab, Pembrolizumab, Cemiplimab, Sintilimab and Toripalimab.

**9.** The pharmaceutical composition for use, the liposomal composition for use, or the PD-1 antagonist for use according to claim 7, wherein the anti-PD-1 antibody is Nivolumab and wherein Nivolumab is administered at 3 mg/kg (body weight) every 2 weeks, at 240 mg every 2 weeks, at 360 mg every 3 weeks or at 480 mg every 4 weeks.

**10.** The pharmaceutical composition for use, the liposomal composition for use, or the PD-1 antagonist for use according to claim 7, wherein Eribulin or the pharmaceutically acceptable salt thereof is Eribulin mesylate and the anti-PD-1 antibody is Nivolumab, wherein Eribulin mesylate is intravenously administered at 1.7 to 2.1 mg / $m^2$ (body surface area) and Nivolumab is intravenously administered at 360 mg on day 1 of a 21-day cycle.

**11.** The pharmaceutical composition for use, the liposomal composition for use, or the PD-1 antagonist for use according to claim 7, wherein Eribulin or the pharmaceutically acceptable salt thereof is Eribulin mesylate and the anti-PD-1 antibody is Nivolumab, wherein Eribulin mesylate is intravenously administered at 1.1 to 1.4 mg / $m^2$ (body surface area) and Nivolumab is intravenously administered at 240 mg on days 1 and 15 of a 28-day cycle.

**12.** The pharmaceutical composition for use, the liposomal composition for use, or the PD-1 antagonist for use according to any one of claims 1 to 11, wherein the tumor is breast cancer, gastric cancer, esophageal cancer, small cell lung cancer, colorectal cancer or renal cancer.

**13.** The pharmaceutical composition for use, the liposomal composition for use, or the PD-1 antagonist for use according to any one of claims 1 to 11, wherein the tumor is breast cancer.

**14.** The pharmaceutical composition for use, the liposomal composition for use, or the PD-1 antagonist for use according

to any one of claims 1 to 11, wherein the tumor is gastric cancer.

15. The pharmaceutical composition for use, the liposomal composition for use, or the PD-1 antagonist for use according to any one of claims 1 to 11, wherein the tumor is esophageal cancer.

16. The pharmaceutical composition for use, the liposomal composition for use, or the PD-1 antagonist for use according to any one of claims 1 to 11, wherein the tumor is small cell lung cancer.

17. The pharmaceutical composition for use, the liposomal composition for use, or the PD-1 antagonist for use according to any one of claims 1 to 11, wherein the tumor is intrahepatic cholangiocarcinoma.

**Patentansprüche**

1. Pharmazeutische Zusammensetzung zur Verwendung bei der Behandlung von Tumoren, umfassend eine liposomale Zusammensetzung, umfassend Eribulin oder ein pharmazeutisch akzeptables Salz davon in der liposomalen inneren Phase,

   wobei die pharmazeutische Zusammensetzung in Kombination mit einem Programmierten-Zelltod-1-Protein (Programmed-Cell-Death-1-Protein; PD-1)-Antagonisten verabreicht wird, und
   wobei der Tumor Brustkrebs, Magenkrebs, Speiseröhrenkrebs, kleinzelliger Lungenkrebs, Darmkrebs, Nierenkrebs, Thymuskarzinom oder intrahepatisches Cholangiokarzinom ist.

2. Pharmazeutische Zusammensetzung zur Verwendung bei der Behandlung von Tumoren, umfassend einen Programmierten-Zelltod-1-Protein (PD-1)-Antagonisten,

   wobei die pharmazeutische Zusammensetzung in Kombination mit einer liposomalen Zusammensetzung verabreicht wird, die Eribulin oder ein pharmazeutisch akzeptables Salz davon in der liposomalen inneren Phase umfasst, und
   wobei der Tumor Brustkrebs, Magenkrebs, Speiseröhrenkrebs, kleinzelliger Lungenkrebs, Darmkrebs, Nierenkrebs, Thymuskarzinom oder intrahepatisches Cholangiokarzinom ist.

3. Liposomale Zusammensetzung, die Eribulin oder ein pharmazeutisch akzeptables Salz davon in der liposomalen inneren Phase umfasst, zur Verwendung bei der Behandlung von Tumoren,

   wobei die liposomale Zusammensetzung in Kombination mit einem PD-1-Antagonisten verabreicht wird, und
   wobei der Tumor Brustkrebs, Magenkrebs, Speiseröhrenkrebs, kleinzelliger Lungenkrebs, Darmkrebs, Nierenkrebs, Thymuskarzinom oder intrahepatisches Cholangiokarzinom ist.

4. PD-1-Antagonist zur Verwendung bei der Behandlung von Tumoren,

   wobei der PD-1-Antagonist in Kombination mit einer liposomalen Zusammensetzung verabreicht wird, die Eribulin oder ein pharmazeutisch akzeptables Salz davon in der liposomalen inneren Phase umfasst, und
   wobei der Tumor Brustkrebs, Magenkrebs, Speiseröhrenkrebs, kleinzelliger Lungenkrebs, Darmkrebs, Nierenkrebs, Thymuskarzinom oder intrahepatisches Cholangiokarzinom ist.

5. Pharmazeutische Zusammensetzung zur Verwendung, die liposomale Zusammensetzung zur Verwendung, oder der PD-1-Antagonist zur Verwendung gemäß mindestens einem der Ansprüche 1 bis 4, wobei die liposomale Zusammensetzung, die Eribulin oder ein pharmazeutisch akzeptables Salz davon in der liposomalen inneren Phase umfasst, und der PD-1-Antagonist, gleichzeitig, getrennt, kontinuierlich oder in einem zeitlichen Abstand verabreicht werden.

6. Pharmazeutische Zusammensetzung zur Verwendung, die liposomale Zusammensetzung zur Verwendung, oder der PD-1-Antagonist zur Verwendung gemäß mindestens einem der Ansprüche 1 bis 5, wobei Eribulin oder das pharmazeutisch akzeptable Salz davon Eribulinmesylat ist.

7. Pharmazeutische Zusammensetzung zur Verwendung, die liposomale Zusammensetzung zur Verwendung, oder der PD-1-Antagonist zur Verwendung gemäß mindestens einem der Ansprüche 1 bis 6, wobei der PD-1-Antagonist

ein Anti-PD-1-Antikörper ist.

8. Pharmazeutische Zusammensetzung zur Verwendung, die liposomale Zusammensetzung zur Verwendung, oder der PD-1-Antagonist zur Verwendung gemäß Anspruch 7, wobei der Anti-PD-1-Antikörper ausgewählt ist, aus der Gruppe, bestehend aus Nivolumab, Pembrolizumab, Cemiplimab, Sintilimab und Toripalimab.

9. Pharmazeutische Zusammensetzung zur Verwendung, die liposomale Zusammensetzung zur Verwendung, oder der PD-1-Antagonist zur Verwendung gemäß Anspruch 7, wobei der Anti-PD-1-Antikörper Nivolumab ist und wobei Nivolumab in einer Dosis von 3 mg/kg (Körpergewicht) alle 2 Wochen, in einer Dosis von 240 mg alle 2 Wochen, in einer Dosis von 360 mg alle 3 Wochen oder in einer Dosis von 480 mg alle 4 Wochen verabreicht wird.

10. Pharmazeutische Zusammensetzung zur Verwendung, die liposomale Zusammensetzung zur Verwendung, oder der PD-1-Antagonist zur Verwendung gemäß Anspruch 7, wobei Eribulin oder dessen pharmazeutisch akzeptables Salz Eribulinmesylat ist und der Anti-PD-1-Antikörper Nivolumab ist, wobei Eribulinmesylat intravenös in einer Dosis von 1,7 bis 2,1 $mg/m^2$ (Körperoberfläche) verabreicht wird und Nivolumab in einer Dosis von 360 mg am Tag 1 eines 21-tägigen Zyklus intravenös verabreicht wird.

11. Pharmazeutische Zusammensetzung zur Verwendung, die liposomale Zusammensetzung zur Verwendung, oder der PD-1-Antagonist zur Verwendung gemäß Anspruch 7, wobei Eribulin oder dessen pharmazeutisch verträgliches Salz Eribulinmesylat ist und der Anti-PD-1-Antikörper Nivolumab ist, wobei Eribulinmesylat intravenös in einer Dosis von 1,1 bis 1,4 $mg/m^2$ (Körperoberfläche) verabreicht wird und Nivolumab in einer Dosis von 240 mg an den Tagen 1 und 15 eines 28-Tage-Zyklus intravenös verabreicht wird.

12. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor Brustkrebs, Magenkrebs, Speiseröhrenkrebs, kleinzelliger Lungenkrebs, Darmkrebs oder Nierenkrebs ist.

13. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor Brustkrebs ist.

14. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor Magenkrebs ist.

15. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor Speiseröhrenkrebs ist.

16. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor kleinzelliger Lungenkrebs ist.

17. Pharmazeutische Zusammensetzung zur Verwendung, die liposomale Zusammensetzung zur Verwendung, oder der PD-1-Antagonist zur Verwendung gemäß mindestens einem der Ansprüche 1 bis 11, wobei der Tumor intrahepatisches Cholangiokarzinom ist.

## Revendications

1. Composition pharmaceutique destinée à être utilisée dans le traitement d’une tumeur, comprenant une composition liposomale comprenant de l’éribuline ou un sel pharmaceutiquement acceptable de celle-ci dans la phase interne liposomale,

    dans laquelle la composition pharmaceutique est administrée en combinaison avec un antagoniste de la protéine 1 de la mort cellulaire programmée (PD-1), et
    dans laquelle la tumeur est un cancer du sein, un cancer gastrique, un cancer de l’œsophage, un cancer du poumon à petites cellules, un cancer colorectal, un cancer du rein, un carcinome thymique ou un cholangiocarcinome intrahépatique.

2. Composition pharmaceutique destinée à être utilisée dans le traitement d'une tumeur, comprenant un antagoniste de la protéine 1 de la mort cellulaire programmée (PD-1),

dans laquelle la composition pharmaceutique est administrée en combinaison avec une composition liposomale comprenant de l'éribuline ou un sel pharmaceutiquement acceptable de celle-ci dans la phase interne liposomale, et
dans laquelle la tumeur est un cancer du sein, un cancer gastrique, un cancer de l'œsophage, un cancer du poumon à petites cellules, un cancer colorectal, un cancer du rein, un carcinome thymique ou un cholangiocarcinome intrahépatique.

3. Composition liposomale comprenant de l'éribuline ou un sel pharmaceutiquement acceptable de celle-ci dans la phase interne liposomale, destinée à être utilisée dans le traitement d'une tumeur,

dans laquelle la composition liposomale est administrée en combinaison avec un antagoniste de PD-1, et
dans laquelle la tumeur est un cancer du sein, un cancer gastrique, un cancer de l'œsophage, un cancer du poumon à petites cellules, un cancer colorectal, un cancer du rein, un carcinome thymique ou un cholangiocarcinome intrahépatique.

4. Antagoniste de PD-1 destiné à être utilisé dans le traitement d'une tumeur,

dans lequel l'antagoniste de PD-1 est administré en combinaison avec une composition liposomale comprenant de l'éribuline ou un sel pharmaceutiquement acceptable de celle-ci dans la phase interne liposomale, et
dans lequel la tumeur est un cancer du sein, un cancer gastrique, un cancer de l'œsophage, un cancer du poumon à petites cellules, un cancer colorectal, un cancer du rein, un carcinome thymique ou un cholangiocarcinome intrahépatique.

5. Composition pharmaceutique destinée à être utilisée, composition liposomale destinée à être utilisée, ou antagoniste de PD-1 destiné à être utilisé selon l'une quelconque des revendications 1 à 4, dans lesquels la composition liposomale comprenant de l'éribuline ou le sel pharmaceutiquement acceptable de celle-ci dans la phase interne liposomale et l'antagoniste de PD-1 sont administrés simultanément, séparément, en continu, ou à un intervalle de temps.

6. Composition pharmaceutique destinée à être utilisée, composition liposomale destinée à être utilisée, ou antagoniste de PD-1 destiné à être utilisé selon l'une quelconque des revendications 1 à 5, dans lesquels l'éribuline ou le sel pharmaceutiquement acceptable de celle-ci est le mésylate d'éribuline.

7. Composition pharmaceutique destinée à être utilisée, composition liposomale destinée à être utilisée, ou antagoniste de PD-1 destiné à être utilisé selon l'une quelconque des revendications 1 à 6, dans lesquels l'antagoniste de PD-1 est un anticorps anti-PD-1.

8. Composition pharmaceutique destinée à être utilisée, composition liposomale destinée à être utilisée, ou antagoniste de PD-1 destiné à être utilisé selon la revendication 7 dans lesquels l'anticorps anti-PD-1 est sélectionné parmi le groupe consistant en Nivolumab, Pembrolizumab, Cemiplimab, Sintilimab et Toripalimab.

9. Composition pharmaceutique destinée à être utilisée, composition liposomale destinée à être utilisée, ou antagoniste de PD-1 destiné à être utilisé selon la revendication 7, dans lesquels l'anticorps anti-PD-1 est Nivolumab et dans lesquels Nivolumab est administré à 3 mg/kg (poids corporel) toutes les 2 semaines, à 240 mg toutes les 2 semaines, à 360 mg toutes les 3 semaines ou à 480 mg toutes les 4 semaines.

10. Composition pharmaceutique destinée à être utilisée, composition liposomale destinée à être utilisée, ou antagoniste de PD-1 destiné à être utilisé selon la revendication 7, dans lesquels l'éribuline ou le sel pharmaceutiquement acceptable de celle-ci est le mésylate d'éribuline et l'anticorps anti-PD-1 est Nivolumab, dans lesquels le mésylate d'éribuline est administré par voie intraveineuse à 1,7 à 2,1 mg / $m^2$ (surface corporelle) et Nivolumab est administré par voie intraveineuse à 360 mg au jour 1 d'un cycle de 21 jours.

11. Composition pharmaceutique destinée à être utilisée, composition liposomale destinée à être utilisée, ou antagoniste

de PD-1 destiné à être utilisé selon la revendication 7, dans lesquels l'éribuline ou le sel pharmaceutiquement acceptable de celle-ci est le mésylate d'éribuline et l'anticorps anti-PD-1 est Nivolumab, dans lesquels le mésylate d'éribuline est administré par voie intraveineuse à 1,1 à 1,4 mg / $m^2$ (surface corporelle) et Nivolumab est administré par voie intraveineuse à 240 mg aux jours 1 et 15 d'un cycle de 28 jours.

**12.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cancer du sein, un cancer gastrique, un cancer de l'œsophage, un cancer du poumon à petites cellules, un cancer colorectal ou un cancer du rein.

**13.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cancer du sein.

**14.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cancer gastrique.

**15.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cancer de l'œsophage.

**16.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cancer du poumon à petites cellules.

**17.** Composition pharmaceutique destinée à être utilisée, composition liposomale destinée à être utilisée, ou antagoniste de PD-1 destiné à être utilisé selon l'une quelconque des revendications 1 à 11, dans lesquels la tumeur est un cholangiocarcinome intrahépatique.

Fig.1

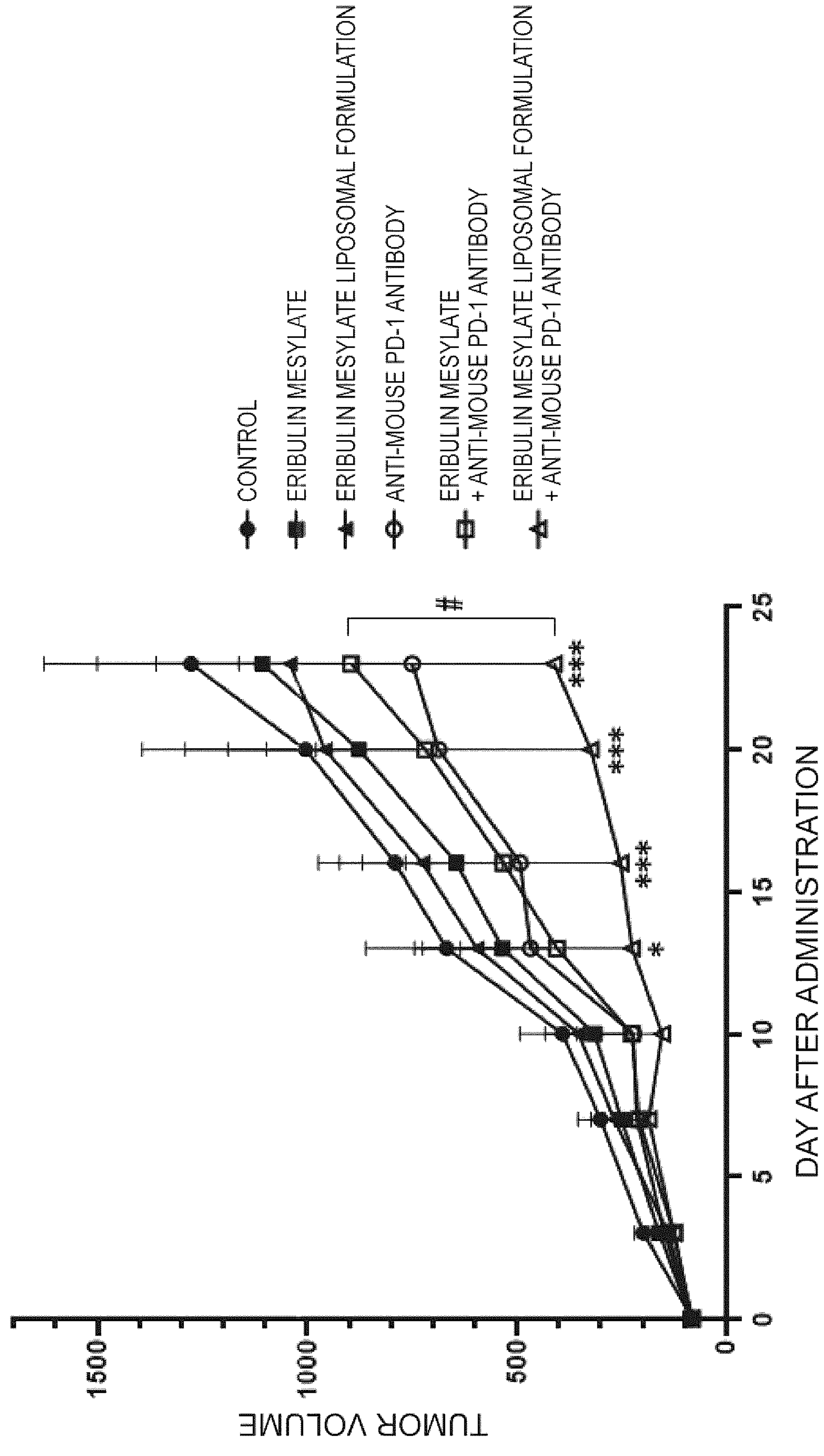
TUMOR VOLUME
0
500
1000
1500
DAY AFTER ADMINISTRATION
0
5
10
15
20
25
*
***
***
***
#
CONTROL
ERIBULIN MESYLATE
ERIBULIN MESYLATE LIPOSOMAL FORMULATION
ANTI-MOUSE PD-1 ANTIBODY
ERIBULIN MESYLATE
+ ANTI-MOUSE PD-1 ANTIBODY
ERIBULIN MESYLATE LIPOSOMAL FORMULATION
+ ANTI-MOUSE PD-1 ANTIBODY

# Fig.2

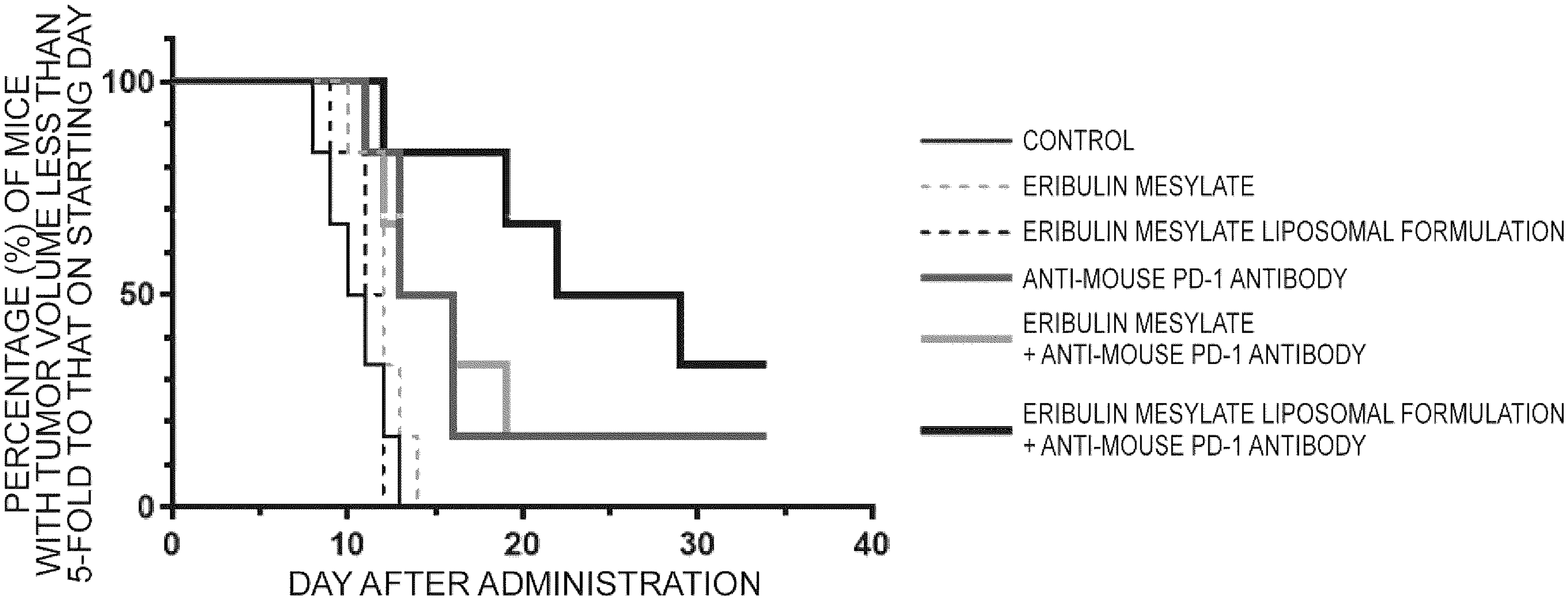
PERCENTAGE (%) OF MICE WITH TUMOR VOLUME LESS THAN 5-FOLD TO THAT ON STARTING DAY
100
50
0
0
10
20
30
40
DAY AFTER ADMINISTRATION
CONTROL
ERIBULIN MESYLATE
ERIBULIN MESYLATE LIPOSOMAL FORMULATION
ANTI-MOUSE PD-1 ANTIBODY
ERIBULIN MESYLATE + ANTI-MOUSE PD-1 ANTIBODY
ERIBULIN MESYLATE LIPOSOMAL FORMULATION + ANTI-MOUSE PD-1 ANTIBODY

Fig.3

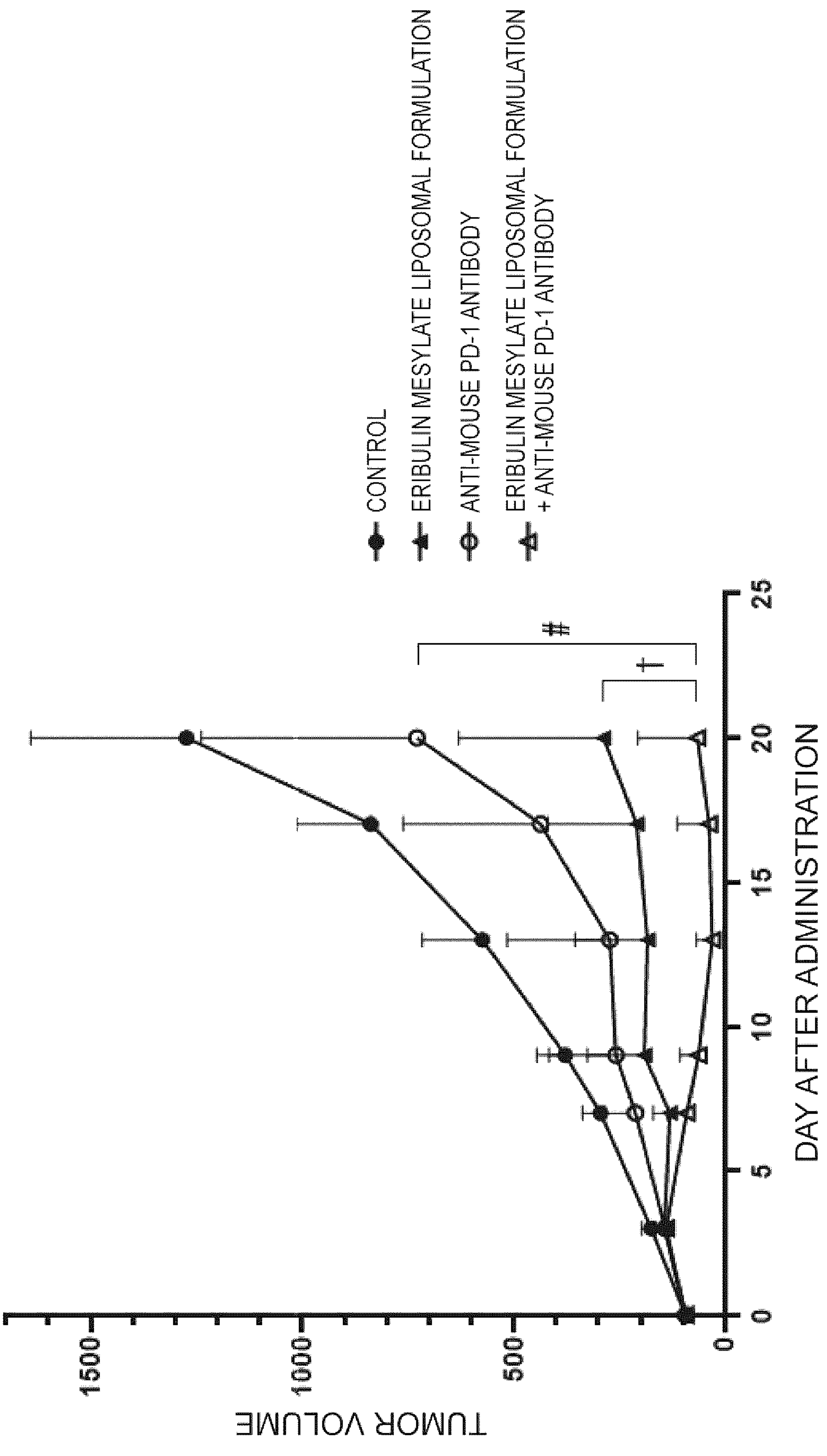
TUMOR VOLUME
1500
1000
500
0
0
5
10
15
20
25
DAY AFTER ADMINISTRATION
†
#
CONTROL
ERIBULIN MESYLATE LIPOSOMAL FORMULATION
ANTI-MOUSE PD-1 ANTIBODY
ERIBULIN MESYLATE LIPOSOMAL FORMULATION
+ ANTI-MOUSE PD-1 ANTIBODY

Fig.4

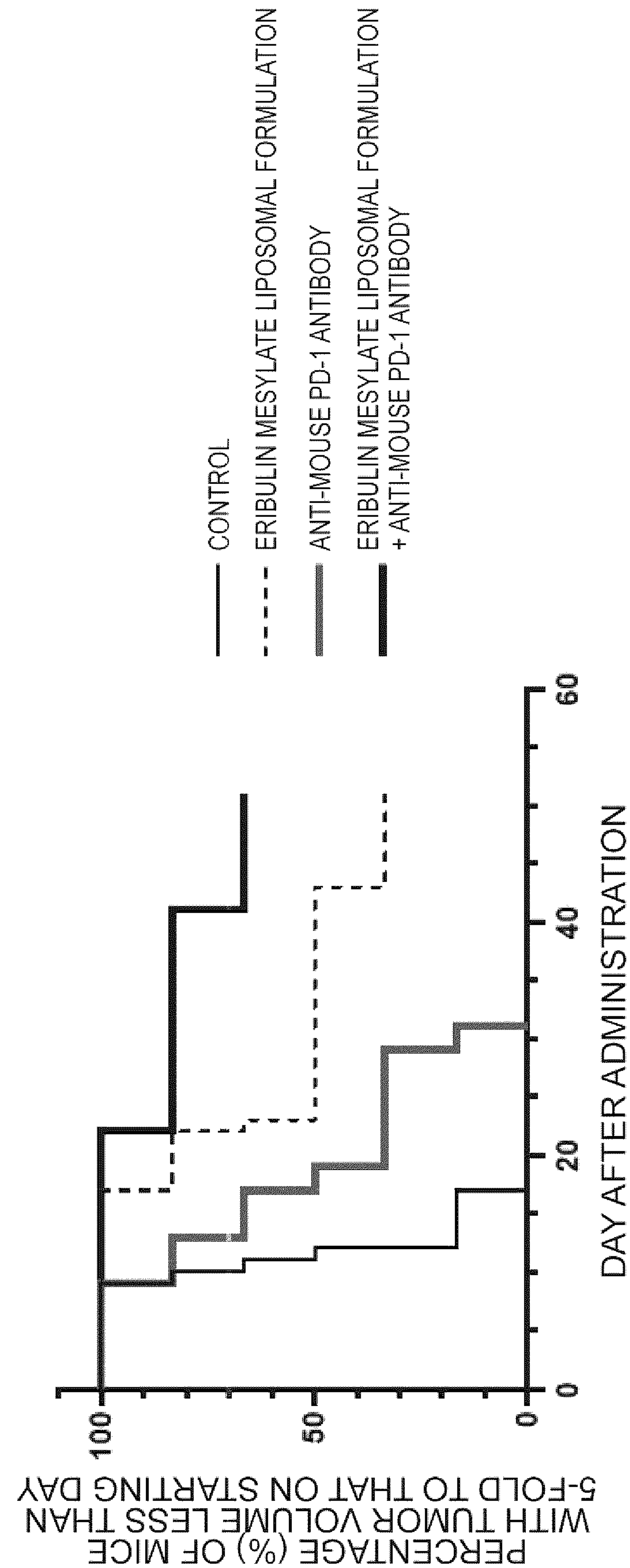
CONTROL
ERIBULIN MESYLATE LIPOSOMAL FORMULATION
ANTI-MOUSE PD-1 ANTIBODY
ERIBULIN MESYLATE LIPOSOMAL FORMULATION + ANTI-MOUSE PD-1 ANTIBODY
PERCENTAGE (%) OF MICE WITH TUMOR VOLUME LESS THAN 5-FOLD TO THAT ON STARTING DAY
100
50
0
0
20
40
60
DAY AFTER ADMINISTRATION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9965894 A
- WO 2005118565 A
- WO 2011094339 A
- US 6469182 B
- US 2006104984
- US 6653341 B
- WO 2010113984 A
- WO 2017188350 A
- WO 2016141209 A
- US 6214865 B
- US 7488802 B
- US 7521051 B
- US 8008449 B
- US 8354509 B
- US 8168757 B
- WO 2004004771 A
- WO 2004072286 A
- WO 2004056875 A
- US 20110271358


### Non-patent literature cited in the description


- **SHARPE, A.H** ; **WHERRY, E.J.** ; **AHMED R.** ; **FREEMAN G.J.** The function of programmed cell death 1 and its ligands in regulating autoimmunity and infection. *Nature Immunology*, 2007, vol. 8, 239-245
- **DONG H et al.** Tumor-associated B7-H1 promotes T-cell apoptosis: a potential mechanism of immune evasion.. *Nat Med.*, August 2002, vol. 8 (8), 793-800
- **YANG et al.** PD-1 interaction contributes to the functional suppression of T-cell responses to human uveal melanoma cells in vitro.. *Invest Ophthalmol Vis Sci.*, June 2008, vol. 49 (6), 2518-2525
- **GHEBEH et al.** The B7-H1 (PD-L1) T lymphocyte-inhibitory molecule is expressed in breast cancer patients with infiltrating ductal carcinoma: correlation with important high-risk prognostic factors.. *Neoplasia*, 2006, vol. 8, 190-198
- **HAMANISHI J et al.** Programmed cell death 1 ligand 1 and tumor-infiltrating CD8+ T lymphocytes are prognostic factors of human ovarian cancer.. *Proceeding of the National Academy of Sciences*, 2007, vol. 104, 3360-3365
- **THOMPSON RH et al.** Significance of B7-H1 overexpression in kidney cancer.. *Clinical genitourin Cancer*, 2006, vol. 5, 206-211
- **NOMI, T.** ; **SHO, M.** ; **AKAHORI, T. et al.** Clinical significance and therapeutic potential of the programmed death- 1 ligand/programmed death-1 pathway in human pancreatic cancer.. *Clinical Cancer Research*, 2007, vol. 13, 2151-2157
- **OHIGASHI Y et al.** Clinical significance of programmed death-1 ligand-1 and programmed death-1 ligand 2 expression in human esophageal cancer. *Clin. Cancer Research*, 2005, vol. 11, 2947-2953
- **INMAN et al.** PD-L1 (B7-H1) expression by urothelial carcinoma of the bladder and BCG-induced granulomata: associations with localized stage progression.. *Cancer*, 2007, vol. 109, 1499-1505
- **SHIMAUCHI T et al.** Augmented expression of programmed death-1 in both neoplasmatic and nonneoplastic CD4+ T-cells in adult T-cell Leukemia/ Lymphoma.. *Int. J. Cancer*, 2007, vol. 121, 2585-2590
- **GAO et al.** Overexpression of PD-L1 significantly associates with tumor aggressiveness and postoperative recurrence in human hepatocellular carcinoma. *Clinical Cancer Research*, 2009, vol. 15, 971-979
- **NAKANISHI J.** Overexpression of B7-H1 (PD-L1) significantly associates with tumor grade and postoperative prognosis in human urothelial cancers.. *Cancer Immunol Immunother.*, 2007, vol. 56, 1173-1182
- **HINO et al.** Tumor cell expression of programmed cell death-1 is a prognostic factor for malignant melanoma.. *Cancer*, 2010, vol. 116, 1757-1766
- **GHEBEH H.** Foxp3+ tregs and B7-H1+/PD-1+ T lymphocytes co-infiltrate the tumor tissues of high-risk breast cancer patients: implication for immunotherapy.. *BMC Cancer.*, 23 February 2008, vol. 8, 57

- **AHMADZADEH M. et al.** Tumor antigen-specific CD8 T cells infiltrating the tumor express high levels of PD-1 and are functionally impaired.. *Blood*, 2009, vol. 114, 1537-1544
- **THOMPSON RH et al.** PD-1 is expressed by tumor infiltrating cells and is associated with poor outcome for patients with renal carcinoma.. *Clinical Cancer Research*, 2007, vol. 15, 1757-1761
- **HIROSHI KIKUCHI et al.** Liposome I -How to prepare and assay. *Cell technology*, 1983, vol. 2 (9), 1136-1149